# EUROPEAN PATENT APPLICATION

(11) **EP 4 793 286 A1**
(43) Date of publication of application: **19.08.2026**
(21) Application number: 24876554.7
(22) Date of filing: 09.10.2024
(51) Int. Cl.: C07K 16/28, C12N 15/13, C12N 15/63, C12N 1/21, A61K 39/395, A61K 48/00

(54) **ANTI-PD-1 MONOCLONAL ANTIBODY AND USE THEREOF**

(30) Priority: 10.10.2023 CN 202311307741; 30.01.2024 CN 202410131767
(71) Applicant: Shanghai Scizeng Medical Technology Co., Ltd., Shanghai 201318 (CN)
(72) Inventor: JIANG, Wenbo, Changchun, Jilin 130012 (CN); XU, John Liuzhong, Changchun, Jilin 130012 (CN); CHU, Xuebin, Changchun, Jilin 130012 (CN); SONG, Lei, Changchun, Jilin 130012 (CN); LI, Xue, Changchun, Jilin 130012 (CN); XUE, Weili, Changchun, Jilin 130012 (CN); LI, Lingyun, Changchun, Jilin 130012 (CN); ZHAO, Ranran, Changchun, Jilin 130012 (CN); HE, Xuzhi, Changchun, Jilin 130012 (CN); WANG, Siqin, Changchun, Jilin 130012 (CN); JIN, Lei, Changchun, Jilin 130012 (CN)
(74) Representative: Kolster Oy Ab
(86) International application number: PCT/CN2024/123741
(87) International publication number: WO 2025/077741

(57) **Abstract**

The present invention relates to the technical field of antibody drugs, and in particular to an anti-PD-1 monoclonal antibody and the use thereof. Specifically, the present invention provides an antibody or an antigen-binding fragment thereof that specifically binds to PD-1, a pharmaceutical composition comprising the antibody or the antigen-binding fragment thereof, a nucleic acid molecule encoding the antibody or the antigen-binding fragment thereof, a host cell containing the nucleic acid molecule, and the therapeutic and diagnostic use of the antibody or the antigen-binding fragment thereof.

## Description

### CROSS-REFERENCE TO RELATED APPLICATIONS

The present application claims priority to Chinese Patent Application No. 202311307741.X filed on October 10, 2023, and Chinese Patent Application No. 202410131767.1 filed on January 30, 2024, which are incorporated herein by reference in their entirety.

### TECHNICAL FIELD

The present invention relates to the technical field of antibody drugs, and in particular to an anti-PD-1 monoclonal antibody and the use thereof.

### BACKGROUND ART

Programmed death-1 (PD-1, also known as CD279) is an inhibitory receptor that is primarily expressed on the surface of activated T cells. Certain subsets of B cells, monocytes, and dendritic cells also express PD-1 after activation.

PD-1 belongs to the immunoglobulin superfamily, has a total length of 288 amino acids, and contains an extracellular domain, a transmembrane domain, and a cytoplasmic domain. The extracellular domain contains an immunoglobulin-like domain (IgV-like domain) responsible for binding to its ligand. The cytoplasmic domain contains an immunoreceptor tyrosine inhibitory motif (ITIM) and an immunoreceptor tyrosine switching motif (ITSM), which are responsible for signal transduction.

PD-1 has two ligands: programmed death ligand 1 (PD-L1) and programmed death ligand 2 (PD-L2). Naïve T cells do not express PD-1 on their surface. When the TCR/CD3 complex and CD28 on the surface of naive T cells bind to MHC-antigen peptides and CD80/CD86, respectively, the intracellular regions of the TCR/CD3 complex and CD28 are phosphorylated by protein kinases, thereby recruiting a series of signal transduction proteins and initiating a series of phosphorylation events, ultimately leading to the activation of naive T cells, and then the surface of the cells begins to upregulate PD-1. When PD-1 binds to PD-L1 or PD-L2 on the surface of antigen-presenting cells, PD-1 is activated, and its cytoplasmic domain is phosphorylated by protein kinases, thus recruiting protein phosphatase SHP-2. SHP-2 can dephosphorylate CD28 and the TCR/CD3 complex intracellularly, thereby inhibiting T cell activation.

As an inhibitory receptor, PD-1 plays an important role in suppressing immune responses and maintaining peripheral tolerance. PD-1 gene defects can lead to the development of autoimmune diseases. C57BL/6 mice with PD-1 gene knockout spontaneously develop lupus-like glomerulonephritis and arthritis symptoms (H Nishimura et al., Immunity. 1999 Aug;11(2):141-51.). NZB/W F1 mice spontaneously develop symptoms similar to those of human systemic lupus erythematosus, and thus serve as the most commonly used mouse model for the study of human systemic lupus erythematosus. Treatment with mouse PD-L1 fusion proteins can alleviate proteinuria symptoms in NZB/W F1 mice and prolong their survival time (Wenjun Liao et al., Am J Nephrol. 2017;46(5):371-379.). Chicken type II collagen can induce arthritis symptoms in mice. Collagen-induced mouse arthritis models are the most commonly used disease models for human rheumatoid arthritis. Treatment with mouse PD-L1 fusion protein can alleviate arthritis symptoms in C57BL/6 mice (Amalia P Raptopoulou et al., Arthritis Rheum. 2010 Jul;62(7):1870-80.).

Although several PD-1 antagonist antibodies that block the PD-1 signaling pathway (such as pembrolizumab, also known as Keytruda) are already on the market and used to treat various tumors, it is still necessary to develop PD-1 agonist antibodies that can activate the PD-1 signaling pathway to suppress T cell responses and thus treat inflammatory and autoimmune diseases.

### SUMMARY OF THE INVENTION

Through extensive research, the inventors of the present application have obtained a murine antibody that specifically binds to PD-1, which has a significant effect on activating the PD-1 signaling pathway. Furthermore, based on the murine antibody, a humanized antibody with PD-1 binding activity and PD-1 signaling pathway activation function has been obtained. The antibody of the present invention can effectively inhibit T cell activity and suppress immune response or inflammatory reaction by activating the PD-1 signaling pathway, thereby playing a role in preventing and treating inflammation, allergies and autoimmune-related diseases. Furthermore, the antibody of the present invention also has cross-species binding activity and activation activity against human and monkey PD-1, which is beneficial for conducting preclinical evaluation studies in animal models.

### Antibody or antigen-binding fragment thereof

In a first aspect, the present application provides an antibody or an antigen-binding fragment thereof that is capable of specifically binding to PD-1, and the antibody or the antigen-binding fragment thereof comprises:
a VH CDR1 or a variant thereof, a VH CDR2 or a variant thereof, and a VH CDR3 or a variant thereof comprised in a heavy chain variable region (VH) set forth in SEQ ID NO: 7 or 15; and/or a VL CDR1 or a variant thereof, a VL CDR2 or a variant thereof, and a VL CDR3 or a variant thereof comprised in a light chain variable region (VL) set forth in SEQ ID NO: 8 or 16;
wherein the variant has one or several amino acid substitutions, deletions or additions (e.g., 1, 2 or 3 amino acid substitutions, deletions or additions, such as conservative substitutions) compared to the sequence from which the variant is derived. In some embodiments, the substitutions are conservative substitutions.

In some embodiments, the antibody or the antigen-binding fragment thereof comprises:
three CDRs comprised in a heavy chain variable region (VH) set forth in SEQ ID NO: 7 or 15; and/or three CDRs comprised in a light chain variable region (VL) set forth in SEQ ID NO: 8 or 16.

In some embodiments, the three CDRs comprised in the VH and/or the three CDRs comprised in the VL are defined by the Kabat, IMGT, or Chothia numbering system.

In some embodiments, the antibody or the antigen-binding fragment thereof comprises:
a heavy chain variable region (VH) comprising the following three complementary determining regions (CDRs): a VH CDR1 set forth in SEQ ID NO: 1, a VH CDR2 set forth in SEQ ID NO: 2, and a VH CDR3 set forth in SEQ ID NO: 3; and/or a light chain variable region (VL) comprising the following three complementary determining regions (CDRs): a VL CDR1 set forth in SEQ ID NO: 4, a VL CDR2 set forth in SEQ ID NO: 5, and a VL CDR3 set forth in SEQ ID NO: 6; wherein the CDR is defined by the Kabat numbering system.

In some embodiments, the antibody or the antigen-binding fragment thereof comprises a framework region sequence derived from a murine immunoglobulin.

In some embodiments, the antibody or the antigen-binding fragment thereof comprises:
a heavy chain variable region (VH) comprising a sequence as set forth in SEQ ID NO: 7 or a variant thereof; and/or a light chain variable region (VL) comprising a sequence as set forth in SEQ ID NO: 8 or a variant thereof;
wherein the variant has one or several amino acid substitutions, deletions or additions (e.g., 1, 2, 3, 4, or 5 amino acid substitutions, deletions or additions), or has at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived. Preferably, the substitutions, deletions, or additions do not exist in the CDR region.

In some embodiments, the substitutions are conservative substitutions.

In some embodiments, the antibody or the antigen-binding fragment thereof comprises: a VH comprising a sequence as set forth in SEQ ID NO: 7 and a VL comprising a sequence as set forth in SEQ ID NO: 8.

In some embodiments, the antibody or the antigen-binding fragment thereof comprises a framework region sequence derived from a human immunoglobulin.

In some embodiments, the antibody or the antigen-binding fragment thereof comprises a framework region comprised in an amino acid sequence encoded by a human germline antibody gene. In some embodiments, the antibody or the antigen-binding fragment thereof comprises: a heavy chain framework region of a human heavy chain germline sequence, and/or a light chain framework region of a human light chain germline sequence. In some embodiments, the heavy chain framework region of the human germline sequence and/or the light chain framework region of the human light chain germline sequence may optionally contain back-mutations from human residues to murine residues.

In some embodiments, the antibody or the antigen-binding fragment thereof comprises: a heavy chain variable region (VH) comprising a sequence as set forth in SEQ ID NO: 15 or a variant thereof; and/or a light chain variable region (VL) comprising a sequence as set forth in SEQ ID NO: 16 or a variant thereof;
wherein the variant has one or several amino acid substitutions, deletions or additions (e.g., 1, 2, 3, 4, or 5 amino acid substitutions, deletions or additions), or has at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived. Preferably, the substitutions, deletions, or additions do not exist in the CDR region.

In some embodiments, the substitutions are conservative substitutions.

In some embodiments, the antibody or the antigen-binding fragment thereof comprises: a heavy chain variable region (VH) comprising a sequence as set forth in SEQ ID NO: 15; and/or a light chain variable region (VL) comprising a sequence as set forth in SEQ ID NO: 16.

In a second aspect, the present application provides an antibody or an antigen-binding fragment thereof that is capable of specifically binding to PD-1, and the antibody or the antigen-binding fragment thereof comprises:
a VH CDR1 or a variant thereof, a VH CDR2 or a variant thereof, and a VH CDR3 or a variant thereof comprised in a heavy chain variable region (VH) set forth in SEQ ID NO: 31 or 39; and/or a VL CDR1 or a variant thereof, a VL CDR2 or a variant thereof, and a VL CDR3 or a variant thereof comprised in a light chain variable region (VL) set forth in SEQ ID NO: 32 or 40;
wherein the variant has one or several amino acid substitutions, deletions or additions (e.g., 1, 2 or 3 amino acid substitutions, deletions or additions, such as conservative substitutions) compared to the sequence from which the variant is derived. In some embodiments, the substitutions are conservative substitutions.

In some embodiments, the antibody or the antigen-binding fragment thereof comprises:
three CDRs comprised in a heavy chain variable region (VH) set forth in SEQ ID NO: 31 or 39; and/or three CDRs comprised in a light chain variable region (VL) set forth in SEQ ID NO: 32 or 40.

In some embodiments, the three CDRs comprised in the VH and/or the three CDRs comprised in the VL are defined by the Kabat, IMGT, or Chothia numbering system.

In some embodiments, the antibody or the antigen-binding fragment thereof comprises:
a heavy chain variable region (VH) comprising the following three complementary determining regions (CDRs): a VH CDR1 set forth in SEQ ID NO: 25, a VH CDR2 set forth in SEQ ID NO: 26, and a VH CDR3 set forth in SEQ ID NO: 27; and/or a light chain variable region (VL) comprising the following three complementary determining regions (CDRs): a VL CDR1 set forth in SEQ ID NO: 28, a VL CDR2 set forth in SEQ ID NO: 29, and a VL CDR3 set forth in SEQ ID NO: 30; wherein the CDR is defined by the Kabat numbering system.

In some embodiments, the antibody or the antigen-binding fragment thereof comprises a framework region sequence derived from a murine immunoglobulin.

In some embodiments, the antibody or the antigen-binding fragment thereof comprises:
a heavy chain variable region (VH) comprising a sequence as set forth in SEQ ID NO: 31 or a variant thereof; and/or a light chain variable region (VL) comprising a sequence as set forth in SEQ ID NO: 32 or a variant thereof;
wherein the variant has one or several amino acid substitutions, deletions or additions (e.g., 1, 2, 3, 4, or 5 amino acid substitutions, deletions or additions), or has at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived. Preferably, the substitutions, deletions, or additions do not exist in the CDR region.

In some embodiments, the substitutions are conservative substitutions.

In some embodiments, the antibody or the antigen-binding fragment thereof comprises: a VH comprising a sequence as set forth in SEQ ID NO: 31 and a VL comprising a sequence as set forth in SEQ ID NO: 32.

In some embodiments, the antibody or the antigen-binding fragment thereof comprises a framework region sequence derived from a human immunoglobulin.

In some embodiments, the antibody or the antigen-binding fragment thereof comprises a framework region comprised in an amino acid sequence encoded by a human germline antibody gene. In some embodiments, the antibody or the antigen-binding fragment thereof comprises: a heavy chain framework region of a human heavy chain germline sequence, and/or a light chain framework region of a human light chain germline sequence. In some embodiments, the heavy chain framework region of the human germline sequence and/or the light chain framework region of the human light chain germline sequence may optionally contain back-mutations from human residues to murine residues.

In some embodiments, the antibody or the antigen-binding fragment thereof comprises: a heavy chain variable region (VH) comprising a sequence as set forth in SEQ ID NO: 39 or a variant thereof; and/or a light chain variable region (VL) comprising a sequence as set forth in SEQ ID NO: 40 or a variant thereof;
wherein the variant has one or several amino acid substitutions, deletions or additions (e.g., 1, 2, 3, 4, or 5 amino acid substitutions, deletions or additions), or has at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived. Preferably, the substitutions, deletions, or additions do not exist in the CDR region.

In some embodiments, the substitutions are conservative substitutions.

In some embodiments, the antibody or the antigen-binding fragment thereof comprises: a heavy chain variable region (VH) comprising a sequence as set forth in SEQ ID NO: 39; and/or a light chain variable region (VL) comprising a sequence as set forth in SEQ ID NO: 40.

In some embodiments, in the first aspect or the second aspect, the antibody or the antigen-binding fragment thereof further comprises a constant region derived from a mammal (e.g., human or mouse) immunoglobulin.

In some embodiments, a heavy chain of the antibody or the antigen-binding fragment thereof comprises a heavy chain constant region derived from a human immunoglobulin (such as IgG, e.g., IgG1, IgG2, IgG3, or IgG4), and/or a light chain of the antibody or the antigen-binding fragment thereof comprises a light chain constant region derived from a human immunoglobulin (e.g., κ or λ).

In some embodiments, the antibody or the antigen-binding fragment thereof comprises a heavy chain constant region with binding activity to Fc receptors (e.g., any one or more of CD32A, CD32B, CD16A).

In some embodiments, the antibody or the antigen-binding fragment thereof comprises a heavy chain constant region (CH) of a wild type human immunoglobulin. In some exemplary embodiments, the heavy chain constant region sequence of the wild-type human immunoglobulin is set forth in SEQ ID NO: 51.

In some embodiments, the antibody or the antigen-binding fragment thereof comprises a light chain constant region (CL) of a wild type human immunoglobulin. In some exemplary embodiments, the light chain constant region sequence of the wild-type human immunoglobulin is set forth in SEQ ID NO: 53.

In some exemplary embodiments, the antibody or the antigen-binding fragment thereof comprises a heavy chain constant region set forth in SEQ ID NO: 51, and/or a light chain constant region set forth in SEQ ID NO: 53.

In some exemplary embodiments, the antibody or the antigen-binding fragment thereof comprises:
(1) a heavy chain comprising a sequence as set forth in SEQ ID NO: 11 and a light chain comprising a sequence as set forth in SEQ ID NO: 12;
(2) a heavy chain comprising a sequence as set forth in SEQ ID NO: 19 and a light chain comprising a sequence as set forth in SEQ ID NO: 20;
(3) a heavy chain comprising a sequence as set forth in SEQ ID NO: 35 and a light chain comprising a sequence as set forth in SEQ ID NO: 36; or,
(4) a heavy chain comprising a sequence as set forth in SEQ ID NO: 43 and a light chain comprising a sequence as set forth in SEQ ID NO: 44.

In some other embodiments, the antibody or the antigen-binding fragment thereof comprises a variant of a heavy chain constant region (CH) of a human immunoglobulin, and the variant of the heavy chain constant region (CH) may comprise one or more amino acid mutations or chemical modifications to change one or more of the following properties of the antibody of the present invention: Fc receptor binding, antibody glycosylation, the number of cysteine residues, effector cell functions or complement functions, etc. Function changes can be produced by replacing at least one amino acid residue in a constant region of an antibody with a different residue or by chemical modification. For example, the affinity of the antibody for an effector ligand (e.g., FcR or complement C1q) is changed, thereby changing effector functions (e.g., reducing or enhancing). An Fc region of an antibody mediates several important effector functions, such as ADCC, phagocytosis, CDC, etc.

In some embodiments, the variant may have enhanced binding ability to Fc receptors (e.g., any one or more of CD32A, CD32B, and CD16A) compared to the wild-type sequence from which it is derived, for example, by at least 1.5-fold, at least 2-fold, or at least 2.5-fold, as detected by surface plasmon resonance technology.

In some embodiments, the variant is a human IgG1 or IgG4 heavy chain constant region variant, which comprises a substitution at one or more of the following positions numbered according to EU: 239 and 268. In some embodiments, the substitution is S239D/H268D. In some embodiments, the substitution is S239D/Q268D.

In some exemplary embodiments, the variant of the heavy chain constant region sequence of the human immunoglobulin has a sequence as set forth in SEQ ID NO: 52.

In some exemplary embodiments, the antibody or the antigen-binding fragment thereof comprises a heavy chain constant region as set forth in SEQ ID NO: 52, and/or a light chain constant region as set forth in SEQ ID NO: 53.

In some exemplary embodiments, the antibody or the antigen-binding fragment thereof comprises:
(1) a heavy chain comprising a sequence as set forth in SEQ ID NO: 23 and a light chain comprising a sequence as set forth in SEQ ID NO: 20; or
(2) a heavy chain comprising a sequence as set forth in SEQ ID NO: 47 and a light chain comprising a sequence as set forth in SEQ ID NO: 44.

In some embodiments, in the first aspect or the second aspect, the antigen-binding fragment is selected from Fab, Fab', F(ab')₂, Fd, Fv, disulfide-linked Fv, scFv, di-scFv, (scFv)₂, a diabody, and a single domain antibody (sdAb); and/or the antibody is a murine antibody, a humanized antibody, a chimeric antibody, a bispecific antibody, or a multispecific antibody.

In a third aspect, the present application further provides an isolated nucleic acid molecule encoding the antibody or the antigen-binding fragment thereof according to the first aspect or the second aspect, or the heavy chain variable region and/or the light chain variable region thereof, or the heavy chain and/or the light chain thereof.

In some embodiments, the isolated nucleic acid molecule comprises a first nucleotide sequence encoding the heavy chain or the heavy chain variable region of the antibody or the antigen-binding fragment thereof of the present invention and a second nucleotide sequence encoding the light chain or the light chain variable region of the antibody or the antigen-binding fragment thereof, wherein the first nucleotide sequence and the second nucleotide sequence are present on the same or different isolated nucleic acid molecules. When the first nucleotide sequence and the second nucleotide sequence are present on different isolated nucleic acid molecules, the isolated nucleic acid molecule of the present invention comprises a first nucleic acid molecule comprising the first nucleotide sequence and a second nucleic acid molecule comprising the second nucleotide sequence.

In some embodiments, the isolated nucleic acid molecule comprises a first nucleotide sequence encoding a heavy chain variable region of the antibody or the antigen-binding fragment of the present invention, and a second nucleotide sequence encoding its light chain variable region, wherein:
(i) the first nucleotide sequence comprises: a sequence as set forth in SEQ ID NO: 9 or a sequence having at least about 85%, 90%, 95%, 99% or higher sequence identity compared with it, or a degenerate sequence thereof; the second nucleotide sequence comprises: a sequence as set forth in SEQ ID NO: 10 or a sequence having at least about 85%, 90%, 95%, 99% or higher sequence identity compared with it, or a degenerate sequence thereof;
(ii) the first nucleotide sequence comprises: a sequence as set forth in SEQ ID NO: 17 or a sequence having at least about 85%, 90%, 95%, 99% or higher sequence identity compared with it, or a degenerate sequence thereof; the second nucleotide sequence comprises: a sequence as set forth in SEQ ID NO: 18 or a sequence having at least about 85%, 90%, 95%, 99% or higher sequence identity compared with it, or a degenerate sequence thereof;
(iii) the first nucleotide sequence comprises: a sequence as set forth in SEQ ID NO: 33 or a sequence having at least about 85%, 90%, 95%, 99% or higher sequence identity compared with it, or a degenerate sequence thereof; the second nucleotide sequence comprises: a sequence as set forth in SEQ ID NO: 34 or a sequence having at least about 85%, 90%, 95%, 99% or higher sequence identity compared with it, or a degenerate sequence thereof; or,
(iv) the first nucleotide sequence comprises: a sequence as set forth in SEQ ID NO: 41 or a sequence having at least about 85%, 90%, 95%, 99% or higher sequence identity compared with it, or a degenerate sequence thereof; the second nucleotide sequence comprises: a sequence as set forth in SEQ ID NO: 42 or a sequence having at least about 85%, 90%, 95%, 99% or higher sequence identity compared with it, or a degenerate sequence thereof.

In some embodiments, the isolated nucleic acid molecule comprises a first nucleotide sequence encoding a heavy chain of the antibody or the antigen-binding fragment of the present invention, and a second nucleotide sequence encoding its light chain, wherein:
(i) the first nucleotide sequence comprises: a sequence as set forth in SEQ ID NO: 13 or a sequence having at least about 85%, 90%, 95%, 99% or higher sequence identity compared with it, or a degenerate sequence thereof; the second nucleotide sequence comprises: a sequence as set forth in SEQ ID NO: 14 or a sequence having at least about 85%, 90%, 95%, 99% or higher sequence identity compared with it, or a degenerate sequence thereof;
(ii) the first nucleotide sequence comprises: a sequence as set forth in SEQ ID NO: 21 or a sequence having at least about 85%, 90%, 95%, 99% or higher sequence identity compared with it, or a degenerate sequence thereof; the second nucleotide sequence comprises: a sequence as set forth in SEQ ID NO: 22 or a sequence having at least about 85%, 90%, 95%, 99% or higher sequence identity compared with it, or a degenerate sequence thereof;
(iii) the first nucleotide sequence comprises: a sequence as set forth in SEQ ID NO: 24 or a sequence having at least about 85%, 90%, 95%, 99% or higher sequence identity compared with it, or a degenerate sequence thereof; the second nucleotide sequence comprises: a sequence as set forth in SEQ ID NO: 22 or a sequence having at least about 85%, 90%, 95%, 99% or higher sequence identity compared with it, or a degenerate sequence thereof;
(iv) the first nucleotide sequence comprises: a sequence as set forth in SEQ ID NO: 37 or a sequence having at least about 85%, 90%, 95%, 99% or higher sequence identity compared with it, or a degenerate sequence thereof; the second nucleotide sequence comprises: a sequence as set forth in SEQ ID NO: 38 or a sequence having at least about 85%, 90%, 95%, 99% or higher sequence identity compared with it, or a degenerate sequence thereof;
(v) the first nucleotide sequence comprises: a sequence as set forth in SEQ ID NO: 45 or a sequence having at least about 85%, 90%, 95%, 99% or higher sequence identity compared with it, or a degenerate sequence thereof; the second nucleotide sequence comprises: a sequence as set forth in SEQ ID NO: 46 or a sequence having at least about 85%, 90%, 95%, 99% or higher sequence identity compared with it, or a degenerate sequence thereof; or,
(vi) the first nucleotide sequence comprises: a sequence as set forth in SEQ ID NO: 48 or a sequence having at least about 85%, 90%, 95%, 99% or higher sequence identity compared with it, or a degenerate sequence thereof; the second nucleotide sequence comprises: a sequence as set forth in SEQ ID NO: 46 or a sequence having at least about 85%, 90%, 95%, 99% or higher sequence identity compared with it, or a degenerate sequence thereof.

In a fourth aspect, the present application further provides a vector comprising the isolated nucleic acid molecule as described above. In some embodiments, the vector is a cloning vector or an expression vector.

In some embodiments, the vector comprises a first nucleotide sequence encoding the heavy chain or the heavy chain variable region of the antibody or the antigen-binding fragment thereof of the present invention and a second nucleotide sequence encoding the light chain or the light chain variable region of the antibody or the antigen-binding fragment thereof, wherein the first nucleotide sequence and the second nucleotide sequence are present on the same or different vectors. When the first nucleotide sequence and the second nucleotide sequence are present on different vectors, the vector of the present invention comprises a first vector comprising the first nucleotide sequence and a second vector comprising the second nucleotide sequence.

In a fifth aspect, the present application further provides a host cell comprising the isolated nucleic acid molecule or the vector as described above.

Such host cells include, but are not limited to, prokaryotic cells such as bacterial cells (e.g., Escherichia coli cells), and eukaryotic cells such as fungal cells (e.g., yeast cells), insect cells, plant cells, and animal cells (e.g., mammalian cells, such as mouse cells and human cells). In some embodiments, the host cells are mammalian cells, such as CHO cells.

The antibody of the present invention can be prepared by various methods known in the art, for example, by a genetic engineering recombination technology. For example, DNA molecules encoding heavy chain and light chain genes of the antibody of the present invention are obtained by chemical synthesis or PCR amplification. The obtained DNA molecules are inserted into an expression vector which is then used for transfecting host cells. Then, the transfected host cells are cultured under specific conditions to express the antibody of the present invention.

The antigen-binding fragments of the present invention can be obtained by hydrolyzing intact antibody molecules (see Morimoto et al., J. Biochem. Biophys. Methods 24:107-117 (1992) and Brennan et al., Science 229:81 (1985)). In addition, these antigen-binding fragments can also be produced directly from recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells. Fab' fragments can be chemically coupled to form F(ab')₂ fragments (Carter et al., Bio/Technology, 10: 163-167 (1992)). In addition, Fv, Fab or F(ab')₂ fragments can also be directly isolated from a culture fluid of recombinant host cells. Other technologies for preparing these antigen-binding fragments are well known to those of ordinary skill in the art.

In a sixth aspect, the present application further provides a method for preparing the antibody or the antigen-binding fragment thereof according to the first aspect or the second aspect. The method comprises the steps of culturing the host cell as described above under conditions allowing the expression of the antibody or the antigen-binding fragment thereof, and recovering the antibody or the antigen-binding fragment thereof from the cultured host cell culture.

### Antibody derivatization

The antibodies or antigen-binding fragments of the present invention can be derivatized, for example, by being linked to another molecule (e.g., another polypeptide or protein). Generally, the derivatization (e.g., labeling) of an antibody or an antigen-binding fragment thereof does not adversely affect its binding to PD-1. Therefore, the antibodies or antigen-binding fragments thereof of the present invention are also intended to include such derivatized forms. For example, the antibody or the antigen-binding fragment thereof of the present invention can be functionally linked (by chemical coupling, gene fusion, non-covalent linkage or other means) to one or more other molecular groups, such as another antibody (e.g., to form a bispecific antibody), a detection reagent, a pharmaceutical reagent, and/or a protein or polypeptide (e.g., avidin or a multihistidine tag) capable of mediating the binding of the antibody or antigen-binding fragment to another molecule.

In a seventh aspect, the present application further provides a bispecific or multispecific molecule which comprises the antibody or the antigen-binding fragment thereof according to the first aspect or the second aspect.

In some embodiments, the bispecific or multispecific molecule specifically binds to PD-1, and additionally specifically binds to one or more other targets.

In some embodiments, the bispecific or multispecific molecule further comprises at least one molecule with a second binding specificity for a second target (e.g., a second antibody).

In an eighth aspect, the present application further provides an immunoconjugate which comprises the antibody or the antigen-binding fragment thereof according to the first aspect or the second aspect and a therapeutic agent linked to the antibody or the antigen-binding fragment thereof.

In some embodiments, the therapeutic agent is selected from cytotoxic agents.

### Pharmaceutical composition

In a ninth aspect, the present application further provides a pharmaceutical composition which comprises the antibody or the antigen-binding fragment thereof according to the first aspect or the second aspect, the isolated nucleic acid molecule according to the third aspect, the vector according to the fourth aspect, the host cell according to the fifth aspect, the bispecific or multispecific molecule according to the seventh aspect, or the immunoconjugate according to the eighth aspect, and a pharmaceutically acceptable carrier and/or excipient.

In some embodiments, the pharmaceutical composition further comprises an additional pharmaceutically active agent.

In some embodiments, the additional pharmaceutically active agent is an immunosuppressant.

In some embodiments, the additional pharmaceutically active agent is a drug for treating autoimmune diseases, allergic diseases, graft-versus-host disease, transplant rejection, or lymphoma (such as T-cell lymphoma).

In some embodiments, in the pharmaceutical composition, the antibody or the antigen-binding fragment thereof, the isolated nucleic acid molecule, the vector, the host cell, the bispecific or multispecific molecule, or the immunoconjugate of the present invention, and the additional pharmaceutically active agent may be provided as separate components or as mixed components. Therefore, the antibody or the antigen-binding fragment thereof, the isolated nucleic acid molecule, the vector, the host cell, the bispecific or multispecific molecule, or the immunoconjugate of the present invention, and the additional pharmaceutically active agent can be administered simultaneously, separately or successively.

In some embodiments, the pharmaceutical composition can comprise a "therapeutically effective amount" or a "prophylactically effective amount" of the antibody or the antigen-binding fragment thereof, the isolated nucleic acid molecule, the vector, the host cell, the bispecific or multispecific molecule, or the immunoconjugate of the present invention. The "prophylactically effective amount" refers to an amount sufficient to prevent, inhibit, or delay the onset of a disease. The "therapeutically effective amount" refers to an amount sufficient to cure or at least partially inhibit a disease and its complications in a patient already suffering from the disease. The therapeutically effective amount may vary depending on the following factors: the severity of the disease to be treated, the overall state of the immune system of the patient, the general conditions of the patient such as age, weight and sex, the manner of administration of a drug, other therapies administered simultaneously, and the like.

### Therapeutic uses

The antibody or the antigen-binding fragment thereof of the present invention can enhance the binding of PD-1 to PD-L1/PD-L2, activate the PD-1 signaling pathway, and thereby inhibit T cell activity. Accordingly, the following therapeutic applications are provided.

In an aspect, the present application provides a method for activating the PD-1 signaling pathway using the antibody or the antigen-binding fragment thereof according to the first aspect or the second aspect, the isolated nucleic acid molecule according to the third aspect, the vector according to the fourth aspect, the host cell according to the fifth aspect, the bispecific or multispecific molecule according to the seventh aspect, the immunoconjugate according to the eighth aspect, or the pharmaceutical composition according to the ninth aspect. In some embodiments, the method includes contacting the antibody or the antigen-binding fragment thereof with cells in vitro to activate the PD-1 signaling pathway in the cells. In some embodiments, the method comprises administering the antibody or the antigen-binding fragment thereof to a subject in need thereof to activate the PD-1 signaling pathway in the subject's body, thereby inhibiting the immune response or inflammatory reaction.

In another aspect, the present application provides the use of the antibody or the antigen-binding fragment thereof according to the first aspect or the second aspect, the isolated nucleic acid molecule according to the third aspect, the vector according to the fourth aspect, the host cell according to the fifth aspect, the bispecific or multispecific molecule according to the seventh aspect, the immunoconjugate according to the eighth aspect, or the pharmaceutical composition according to the ninth aspect in the preparation of PD-1 signaling pathway activators.

In an aspect, the present application provides a method for inhibiting the activity of immune cells using the antibody or the antigen-binding fragment thereof according to the first aspect or the second aspect, the isolated nucleic acid molecule according to the third aspect, the vector according to the fourth aspect, the host cell according to the fifth aspect, the bispecific or multispecific molecule according to the seventh aspect, the immunoconjugate according to the eighth aspect, or the pharmaceutical composition according to the ninth aspect. In some embodiments, the method comprises contacting the antibody or the antigen-binding fragment thereof with cells (such as T cells) in vitro to inhibit their immune activity. In some embodiments, the method comprises administering the antibody or the antigen-binding fragment thereof to a subject in need thereof to inhibit the activity of immune cells in the subject's body, thereby inhibiting the immune response or inflammatory reaction.

In another aspect, the present application provides the use of the antibody or the antigen-binding fragment thereof according to the first aspect or the second aspect, the isolated nucleic acid molecule according to the third aspect, the vector according to the fourth aspect, the host cell according to the fifth aspect, the bispecific or multispecific molecule according to the seventh aspect, the immunoconjugate according to the eighth aspect, or the pharmaceutical composition according to the ninth aspect in the preparation of immunosuppressants (such as T-cell activity inhibitors).

In an aspect, the present application further provides a method for preventing and/or treating a disease in a subject, comprising: administering to a subject in need thereof an effective amount of the antibody or the antigen-binding fragment thereof according to the first aspect or the second aspect, the isolated nucleic acid molecule according to the third aspect, the vector according to the fourth aspect, the host cell according to the fifth aspect, the bispecific or multispecific molecule according to the seventh aspect, the immunoconjugate according to the eighth aspect, or the pharmaceutical composition according to the tenth aspect.

In another aspect, the present application provides the use of the antibody or the antigen-binding fragment thereof, the isolated nucleic acid molecule, the vector, the host cell, the bispecific or multi-specific molecule, the immunoconjugate or the pharmaceutical composition in the preparation of a drug for preventing and/or treating a disease in a subject.

In some embodiments, the disease will benefit from the activation of the PD-1 signaling pathway. In some embodiments, disease is characterized by the inactivation of the PD-1/PD-L1 signaling pathway. In some embodiments, the disease will benefit from the inhibition of the immune response or inflammatory reaction.

In some embodiments, the disease is selected from autoimmune disorders, allergic diseases, graft-versus-host disease, transplant rejection, lymphoma, or any combination thereof.

In some embodiments, the autoimmune disease is selected from arthritis, rheumatoid arthritis, psoriatic arthritis, lupus, lupus nephritis, systemic lupus erythematosus, psoriasis, vitiligo, alopecia areata, inflammatory bowel disease, ulcerative colitis, Crohn's disease, type I diabetes, multiple sclerosis, autoimmune hepatitis, primary biliary cirrhosis, celiac disease, scleroderma, Graves' disease, Hashimoto's thyroiditis, ankylosing spondylitis, myasthenia gravis, Sjögren's syndrome, IgA nephropathy, IgG4-related disease, vasculitis, ANCA-associated vasculitis, uveitis, pemphigus, pemphigoid, or autoimmune hemolytic anemia.

In some embodiments, the allergic disease is selected from idiopathic thrombocytopenic purpura, atopic dermatitis, asthma, allergic rhinitis, drug allergy, food allergy, anaphylaxis, allergic conjunctivitis, urticaria, eosinophilic sinusitis, eosinophilic gastrointestinal disease, or allergic bronchopulmonary aspergillosis.

In some embodiments, the lymphoma is T-cell lymphoma.

In some embodiments, the subject in any of the above aspects is a mammal. In some embodiments, the subject is a human. In some embodiments, the subject is a non-human primate, e.g., a cynomolgus monkey.

In some embodiments, the antibody or the antigen-binding fragment thereof, the isolated nucleic acid molecule, the vector, the host cell, the bispecific or multispecific molecule, the immunoconjugate, or the pharmaceutical composition in the any of the above aspects is used alone, or in combination with other pharmaceutically active agents, for example, administered simultaneously or sequentially. In some embodiments, the additional pharmaceutically active agent is an immunosuppressant. In some embodiments, the additional pharmaceutically active agent is a drug for treating autoimmune diseases, allergic diseases, graft-versus-host disease, transplant rejection, or lymphoma (such as T-cell lymphoma).

In any of the above aspects, the antibody or the antigen-binding fragment thereof or the pharmaceutical composition of the present application can be formulated into any dosage form known in the medical arts, for example, tablets, pills, suspensions, emulsions, solutions, gels, capsules, powders, granules, elixirs, lozenges, suppositories, injections (including injection solutions, sterile powders for injection, and concentrated solutions for injection), inhalants, sprays, etc. The preferred dosage form depends on the intended mode of administration and therapeutic use. The antibody or the antigen-binding fragment thereof or the pharmaceutical composition of the present invention should be sterile and stable under the conditions of production and storage. A preferred dosage form is an injection. Such injections may be sterile injection solutions. For example, the sterile injection solutions can be prepared by incorporating a necessary dose of the antibody or the antigen-binding fragment thereof of the present invention in an appropriate solvent, and optionally, incorporating other desired ingredients (including but not limited to pH adjusting agents, surfactants, adjuvants, ionic strength enhancers, isotonic agents, preservatives, diluents, or any combination thereof), followed by filtration sterilization. Additionally, the sterile injectable solutions can be prepared as sterile lyophilized powders (e.g., by vacuum drying or freeze drying) for ease of storage and use. Such sterile lyophilized powders can be dispersed in suitable carriers before use, such as water for injection (WFI), bacteriostatic water for injection (BWFI), a sodium chloride solution (e.g., 0.9% NaCl), a glucose solution (e.g., 5% glucose), a solution comprising a surfactant (e.g., 0.01% polysorbate 20), a pH buffer (e.g., phosphate buffer), a Ringer's solution, and any combination thereof.

In any of the above aspects, the antibody or the antigen-binding fragment thereof or the pharmaceutical composition of the present application can be administered by any suitable method known in the art, including but not limited to, oral, buccal, sublingual, ocular, topical, parenteral, rectal, intrathecal, intracytoplasmic reticulum, inguinal, intravesical, topical (e.g., powders, ointments or drops), or nasal routes. However, for many therapeutic uses, the preferred route/mode of administration is parenteral administration (e.g., intravenous or bolus injection, subcutaneous injection, intraperitoneal injection, and intramuscular injection). It will be appreciated by technicians that the route and/or mode of administration will vary depending upon the intended purpose. In some embodiments, the antibody or the antigen-binding fragment thereof or the pharmaceutical composition of the present invention is administered by intravenous injection or bolus injection.

### Detection uses

The antibody or the antigen-binding fragment of the present invention can specifically bind to PD-1, thereby providing the following detection applications.

In an aspect, the present application provides a conjugate which comprises the antibody or the antigen-binding fragment thereof according to the first aspect or the second aspect and a detectable label linked to the antibody or the antigen-binding fragment thereof.

In some embodiments, the detectable label is selected from an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

In another aspect, the present application provides a kit which comprises the antibody or the antigen-binding fragment thereof according to the first aspect or the second aspect, or the conjugate as described above.

In some embodiments, the kit comprises the conjugate as described above.

In some embodiments, the kit comprises the antibody or the antigen-binding fragment thereof according to the first aspect or the second aspect, and a second antibody that specifically recognizes the antibody or the antigen-binding fragment thereof. Optionally, the second antibody further comprises a detectable label, such as an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

In another aspect, the present application further provides a method for detecting the presence or level of PD-1 in a sample, which comprises using the antibody or the antigen-binding fragment thereof according to the first aspect or the second aspect or the conjugate as described above.

In some embodiments, the method is used for therapeutic purposes, diagnostic purposes, or non-therapeutic and non-diagnostic purposes.

In some embodiments, the method is an immunological assay, such as an immunoblot, an enzyme immunoassay (e.g., ELISA), a chemiluminescent immunoassay, a fluorescent immunoassay, or a radioimmunoassay.

In some embodiments, the method comprises: (1) contacting the sample with the antibody or the antigen-binding fragment thereof of the present invention; and (2) detecting the formation of an antigen-antibody immune complex, or detecting the amount of the immune complex. The formation of the immune complex indicates the presence of PD-1 or cells expressing PD-1. In some embodiments, the antibody or the antigen-binding fragment thereof carries a detectable label, or a second antibody labeled with a detectable label is used for detecting the antibody or the antigen-binding fragment thereof.

In another aspect, the present application further provides a method for diagnosing a PD-1-related disease, which comprises detecting the levels of PD-1 in a sample from a subject using the method as described above. In some embodiments, the method further comprises comparing the PD-1 level in a sample from a subject with a reference value (e.g., healthy control) to diagnose whether the subject has the disease.

In some embodiments, the PD-1-related disease is characterized by the inactivation of the PD-1/PD-L1 signaling pathway. When the level of PD-1 decreases compared to a reference value (e.g., healthy control), it indicates that the subject has the PD-1-related disease. In some embodiments, the PD-1-related disease includes autoimmune disorders, allergic diseases, graft-versus-host disease, transplant rejection, lymphoma, or combinations thereof. In some embodiments, the lymphoma is T-cell lymphoma.

In some embodiments, the PD-1-related disease is characterized by the excessive activation of the PD-1/PD-L1 signaling pathway. When the level of PD-1 increases compared to a reference value (e.g., healthy control), it indicates that the subject has the PD-1-related disease. In some embodiments, the PD-1-related disease is a tumor.

In some embodiments, the sample may be selected from urine, blood, serum, plasma, saliva, ascites, circulating cells, circulating tumor cells, non-tissue-associated cells (i.e., free cells), tissue (e.g., surgically removed tumor tissue, biopsy sections, or fine-needle aspiration tissue), histological preparations, etc.

In some embodiments, the subject is a mammal, such as a human or a non-human primate (e.g., cynomolgus monkey).

In another aspect, the present application further provides the use of the antibody or the antigen-binding fragment thereof according to the first aspect or the second aspect or the conjugate as described above in the preparation of a detection reagent. The detection reagent is used for detecting the presence or level of PD-1 in a sample and/or diagnosing a PD-1-related disease.

In some embodiments, the detection reagent is used for detecting the presence or level of PD-1 in a sample by the method as described above.

In some embodiments, the detection reagent is used for detecting the presence or level of PD-1 in a sample by the method as described above to diagnose a PD-1-related disease.

### Definitions of terms

In the present invention, unless otherwise specified, the scientific and technical terms used herein have the meanings commonly understood by those skilled in the art. Furthermore, the cell culture, molecular biology, biochemistry, nucleic acid chemistry, immunology and other operation procedures used herein are all common steps widely employed in their respective fields. Moreover, in order to better understand the present invention, definitions and explanations of relevant terms are provided below.

When the terms "for example", "e.g.", "such as", "including", "comprising", or variations thereof are used herein, these terms will not be considered as restrictive terms, but will be interpreted to mean "but not limited to" or "not limited to".

The terms "a", "an", "the", and similar referents in the context of describing the present invention (especially in the context of the following claims) should be construed to cover both the singular and the plural referents, unless otherwise indicated herein or clearly contradicted by context.

As used herein, the term "programmed death-1 (PD-1)" refers to an inhibitory receptor that is primarily expressed on the surface of activated T cells, which is responsible for signal transduction. Activation of the PD-1 signaling pathway can inhibit the activity of T cells. The sequence of the human PD-1 protein-coding gene can be found in NCBI accession number: NM_005018.3.

The term "antibody" as used herein refers to an immunoglobulin-derived molecule capable of specifically binding to a target antigen, and the immunoglobulin-derived molecule binds to the target antigen through at least one antigen-binding site located in a variable region thereof. When referring to the term "antibody", unless otherwise specified in the context, it includes not only an intact antibody but also an antigen-binding fragment capable of specifically binding to a target antigen. An "intact antibody" typically consists of two pairs of polypeptide chains, each pair having one light chain (LC) and one heavy chain (HC). Light chains of the antibody can be classified as kappa (κ) and lambda (λ) light chains. Heavy chains can be classified as µ, δ, γ, α or ε, and the isotype of the antibody is respectively defined as IgM, IgD, IgG, IgA, and IgE. Within light and heavy chains, the variable and constant regions are joined by a "J" region of about 12 or more amino acids, and the heavy chain also comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant domain is not directly involved in binding of an antibody to an antigen, but exhibits various effector functions, such as mediating the binding of immunoglobulins to host tissues or factors, including binding of various cells (e.g., effector cells) of the immune system and the first component (C1q) of the classical complement system. VH and VL regions can be further subdivided into regions with high variability (called complementarity determining regions (CDRs)) interspersed with relatively conserved regions called framework regions (FRs). Each of the VH and VL consists of 3 CDRs and 4 FRs arranged from an amino terminal to a carboxyl terminal in the following order: FR1, CDR1, FR2, CDR2, FR3, CDR3, and FR4. The variable regions (VH and VL) of each heavy chain/light chain pair respectively form an antigen-binding site. The distribution of amino acids in each region or domain can be based on the definitions in Kabat, Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

As used herein, the term "complementarity determining region" or "CDR" refers to amino acid residues responsible for antigen binding in a variable region of an antibody. Each of the variable regions of the heavy and light chains comprises three CDRs, named CDR1, CDR2, and CDR3. The precise boundaries of these CDRs can be defined according to various numbering systems known in the art, such as the definitions in the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given antibody, a person of skill in the art will readily identify the CDRs defined by each numbering system. Furthermore, the corresponding relationship between different numbering systems is well known to a person of skill in the art (for example, see Lefranc et al., Dev. Comparat. Immunol. 27:55-77, 2003).

In the present invention, the CDR comprised in the antibody or the antigen-binding fragment thereof of the present invention can be determined according to various numbering systems known in the art. In some embodiments, the CDR comprised in the antibody or antigen-binding fragment thereof of the present invention is preferably determined by the Kabat numbering system.

As used herein, the term "framework region" or "FR" residues refer to those amino acid residues in variable regions of an antibody other than the CDR residues as defined above.

The term "antibody" is not limited to any particular method of producing the antibody. For example, antibodies include recombinant antibodies, monoclonal antibodies, and polyclonal antibodies. Antibodies can be of different isotypes, for example, IgG (e.g., IgG1, IgG2, IgG3, or IgG4 subtype), IgA1, IgA2, IgD, IgE, or IgM antibodies.

As used herein, the term "antigen-binding fragment" of an antibody refers to a polypeptide comprising a fragment of a full-length antibody that retains the ability to specifically bind to the same antigen that the full-length antibody binds to and/or competes with the full-length antibody for specific binding to the antigen, which is also referred to as an "antigen-binding portion". Generally, see Fundamental Immunology, Ch. 7 (Paul, W., ed., 2nd ed., Raven Press, N.Y. (1989), which is incorporated herein by reference in its entirety for all purposes. The antigen-binding fragment of an antibody can be produced by recombinant DNA technologies or by enzymatic or chemical cleavage of an intact antibody. Non-limiting examples of the antigen-binding fragment include Fab, Fab', F(ab')₂, Fd, Fv, disulfide-linked Fv, scFv, di-scFv, a diabody, a single-domain antibody, and a polypeptide comprising at least a portion of an antibody sufficient to confer a specific antigen-binding ability to the polypeptide. Engineered antibody variants are reviewed in Holliger et al., 2005; Nat Biotechnol, 23: 1126-1136.

As used herein, the term "Fd" means an antibody fragment consisting of VH and CH1 domains; the term "Fab fragment" means an antibody fragment consisting of VL, VH, CL, and CH1 domains; the term "F(ab')₂ fragment" means an antibody fragment comprising two Fab fragments linked by a disulfide bridge on a hinge region; and the term "Fab' fragment" means a fragment obtained after reducing disulfide bonds linking two heavy chain fragments in the F(ab')₂ fragment, consisting of a complete light chain and the Fd fragment (consisting of VH and CH1 domains) of a heavy chain.

As used herein, the term "Fv" means an antibody fragment consisting of the VL and VH domains of a single arm of an antibody. The Fv fragment is generally considered to be the smallest antibody fragment that capable of forming a complete antigen-binding site. It is generally believed that six CDRs confer an antigen-binding specificity to an antibody. However, a single variable region (e.g., the Fd fragment, which comprises only three CDRs specific for an antigen) also has the ability to recognize and bind to an antigen, although the affinity thereof may be lower than that of a complete binding site.

As used herein, the term "Fc" means an antibody fragment formed by the second and third constant regions of a first heavy chain of an antibody binding to the second and third constant regions of a second heavy chain of the antibody through disulfide bonds. The Fc fragment of an antibody has a variety of different functions but is not involved in antigen binding. The "effector functions" mediated by the Fc domain comprise Fc receptor binding; Clq binding and complement-dependent cytotoxicity (CDC); antibody-dependent cell-mediated cytotoxicity (ADCC); phagocytosis; downregulation of cell surface receptors (e.g., B cell receptor); and B cell activation, or the like. The Fc domain may comprise both a native Fc region and a variant Fc region. A native Fc region comprises an amino acid sequence that is consistent with the amino acid sequence of an Fc region found in nature, for example, a native sequence human Fc region comprises a native sequence human IgG1 Fc region; a native sequence human IgG2 Fc region; a native sequence human IgG3 Fc region; and a native sequence human IgG4 Fc region, as well as naturally occurring variants thereof. A variant Fc region comprises an amino acid sequence which differs from the amino acid sequence of a native sequence Fc region by at least one amino acid modification. In some embodiments, a variant Fc region may possess altered effector functions (e.g., Fc receptor binding, antibody glycosylation, number of cysteine residues, effector cell function, or complement function) compared to a native Fc region.

As used herein, the term "scFv" refers to a single polypeptide chain comprising VL and VH domains, wherein the VL and VH are linked by a linker. Such scFv molecules may have a general structure: NH₂-VL-Linker-VH-COOH or NH₂-VH-Linker-VL-COOH. A suitable linker in the prior art consists of repeated GGGGS amino acid sequences or variants thereof. For example, a linker having an amino acid sequence (GGGGS)₄ may be used, but a variant thereof may also be used. In some cases, a disulfide bond may also exist between the VH and VL of the scFv. In some embodiments of the present invention, the scFv may form di-scFv, which refers to two or more single scFvs in series to form an antibody. In some embodiments of the present invention, the scFv may form a (scFv)₂, which refers to two or more single scFvs in parallel to form an antibody.

As used herein, the term "diabody" means that the VH and VL domains thereof are expressed on a single polypeptide chain, but it uses a linker that is too short to allow pairing between two domains of the same chain, thereby forcing the domains to pair with the complementary domains of another chain and forming two antigen-binding sites (see, e.g. Holliger P. et al., Proc. Natl. Acad. Sci. USA 90:6444-6448 (1993), and Poljak R.J. et al., Structure 2:1121-1123 (1994)).

As used herein, the term "single-domain antibody (sdAb)" has the meaning commonly understood by a person of skill in the art, and refers to an antibody fragment consisting of a single monomeric variable antibody domain (e.g., single heavy chain variable region), which retains the ability to specifically bind to the same antigen as the full-length antibody.

As used herein, the term "bispecific antibody" refers to an antibody having a binding specificity for two different antigens (or epitopes). The term "multispecific antibody" refers to an antibody having a binding specificity for at least more than two (e.g., three or four) different antigens (or epitopes). The bispecific antibody or the multispecific antibody comprises multiple antigen-binding domains having a binding specificity for different antigens (or epitopes), and thus is able to bind to at least two different binding sites and/or target molecules. Each antigen-binding domain comprised in the bispecific antibody or the multispecific antibody can be independently selected from a full-length antibody (e.g., IgG antibody) or an antigen-binding fragment thereof (e.g., Fv fragment, Fab fragment, F(ab')₂ fragment, or scFv). In some cases, individual antigen-binding domains are linked by peptide linkers.

Each of the above antibody fragments retains the ability to specifically bind to the same antigen as the full-length antibody, and/or competes with the full-length antibody for specific binding to the antigen.

An antigen-binding fragment of an antibody (e.g., the above-mentioned antibody fragment) can be obtained from a given antibody (e.g., antibody provided in the present invention) by a conventional technology known to those skilled in the art (e.g., recombinant DNA technology or enzymatic or chemical cleavage method), and the antigen-binding fragment of the antibody can be screened for specificity in the same manner as for an intact antibody.

As used herein, the term "humanized antibody" refers to a non-human antibody that is genetically engineered, and the amino acid sequence thereof is modified to increase the homology with the sequence of a human antibody. Generally speaking, all or part of CDR regions of a humanized antibody are derived from a non-human antibody (donor antibody), and all or part of non-CDR regions (e.g., variable region FR and/or constant region) are derived from a human immunoglobulin (receptor antibody). In some embodiments, the CDR regions of a humanized antibody are derived from a non-human antibody (donor antibody), and all or part of non-CDR regions (e.g., variable region FR and/or constant region) are derived from a human immunoglobulin (receptor antibody). A humanized antibody generally retains the desired properties of a donor antibody, including but not limited to, antigen specificity, affinity, reactivity, etc. In the present application, the donor antibody may be a murine antibody having desired properties (e.g., antigen specificity, affinity, reactivity). To prepare a humanized antibody, CDR regions of a donor antibody can be inserted into human framework sequences by a method known in the art. In some cases, the human framework sequence may comprise amino acid mutations that are substituted with corresponding non-human residues. In addition, a humanized antibody may also comprise residues that are not found in a variable region (e.g., light chain variable region or heavy chain variable region) of an initial donor antibody or in a human framework sequence to further improve or optimize the performance of the humanized antibody.

As used herein, the term "chimeric antibody" refers to an antibody in which a portion of the light chain and/or the heavy chain thereof is derived from one antibody (which may be derived from a particular species or belong to a particular antibody type or subtype), and another portion of the light chain and/or the heavy chain is derived from another antibody (which may be derived from the same or different species or belong to the same or different antibody types or subtypes), but in any case, the binding activity to a target antigen is still retained. In some embodiments, the term "chimeric antibody" may include an antibody in which the heavy chain variable region and light chain variable region of the antibody are derived from a first antibody, and the heavy chain constant region and light chain constant region of the antibody are derived from a second antibody.

As used herein, the term "identity" is used for referring to the matching situation of sequences between two polypeptides or between two nucleic acids. To determine the percent identity between two amino acid sequences or two nucleic acid sequences, the sequences are aligned for optimal comparison purposes (for example, gaps can be introduced in a first amino acid or nucleic acid sequence for optimal alignment with a second amino acid or nucleic acid sequence). Then, amino acid residues or nucleotides at corresponding amino acid positions or nucleotide positions are compared. When a position in the first sequence is occupied by the same amino acid residue or nucleotide as the corresponding position in the second sequence, molecules are identical at that position. The percent identity between two sequences is a function of the number of identical positions shared by the sequences (i.e., percent identity = number of identical overlapping positions/total number of positions x 100%). In some embodiments, two sequences have the same length.

The assay of the percent identity between two sequences can also be achieved by a mathematical algorithm. A non-limiting example of a mathematical algorithm for comparison of two sequences is improved in algorithms of Karlin and Altschul, 1990, Proc. Natl. Acad. Sci. U.S.A. 87:2264-2268, as in Karlin and Altschul, 1993, Proc. Natl. Acad. Sci. U.S.A. 90:5873-5877. Such an algorithm is integrated into NBLAST and XBLAST programs of Altschul et al., 1990, J. Mol. Biol. 215:403.

As used herein, the term "variant", in the context of polypeptides (including polypeptides), also refers to a polypeptide or peptide comprising an amino acid sequence that has been altered by the introduction of amino acid residue substitutions, deletions or additions. In some cases, the term "variant" also refers to a polypeptide or peptide that has been modified (i.e., by covalent linkage of any type of molecule to the polypeptide or peptide). For example, but not limitation, the polypeptide can be modified, for example, by glycosylation, acetylation, pegylation, phosphorylation, amidation, derivatization by known protecting/blocking groups, proteolytic cleavage, linkage to cellular ligands or other proteins, etc. Derived polypeptides or peptides can be produced by chemical modification using technologies known to a person of skill in the art. The technologies include but not limited to specific chemical cleavage, acetylation, formylation, metabolic synthesis of tunicamycin, etc. Furthermore, the variant has similar, identical or improved functions as the polypeptide or peptide from which it is derived.

As used herein, the term "specific binding" refers to a non-random binding reaction between two molecules, such as the reaction between an antibody and a targeted antigen. The strength or affinity of a specific binding interaction can be expressed in terms of an equilibrium dissociation constant (K_{D}) for the interaction. In the present invention, the term "K_{D}" refers to a dissociation equilibrium constant for a specific antibody-antigen interaction, which is used for describing the binding affinity between an antibody and an antigen. The smaller the equilibrium dissociation constant, the tighter the antibody-antigen binding, and the higher the affinity between the antibody and the antigen.

The specific binding properties between two molecules can be determined by a method known in the art. One method involves measurement of the rates of formation and dissociation of antigen-binding sites/antigen complexes. Both the "association rate constant" (ka or kon) and the "dissociation rate constant" (kdis or koff) can be calculated by the concentration and the actual rates of association and dissociation. The ratio of kdis/kon is equal to the dissociation constant K_{D}. The values of the K_{D}, kon and kdis can be measured by any effective method. In some embodiments, the dissociation constant can be measured in Biacore by surface plasmon resonance (SPR).

As used herein, the detectable label of the present invention may be any substance detectable by fluorescent, spectroscopic, photochemical, biochemical, immunological, electrical, optical or chemical means. Such labels are well known in the art, and examples thereof include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluoresceins, tetramethylrhodamine isothiocyanate (TRITC), fluorescent proteins (e.g., phycoerythrin (PE)), Texas Red, rhodamine, quantum dots or cyanine dye derivatives (e.g., Cy7 and Alexa 750)), luminescent substances (e.g., chemiluminescent reagents, such as acridinium ester compounds, luminol and derivatives thereof, and ruthenium derivatives such as terpyridine ruthenium), magnetic beads (e.g., Dynabeads^{®}), calorimetric labels such as colloidal gold or colored glass or plastic (e.g., polystyrene, polypropylene, latex, etc.) beads, and biotins for binding to avidins (e.g., streptavidin) modified with the above labels. In some embodiments, the detectable label is selected from an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin. In some embodiments, the detectable label described above can be linked to the antibody or the antigen-binding fragment thereof of the present invention through linkers of different lengths to reduce potential steric hindrance.

As used herein, the term "vector" refers to a nucleic acid delivery vehicle into which a polynucleotide can be inserted. When the vector can express the protein encoded by the inserted polynucleotide, the vector is called an expression vector. The vector can be introduced into the host cell by transformation, transduction or transfection, so that the genetic material elements carried by the vector are expressed in the host cell. Vectors are well known to those skilled in the art, and include but are not limited to: plasmids; bacteriophagemids; cosmids; artificial chromosomes, such as yeast artificial chromosomes (YACs), bacterial artificial chromosomes (BACs), or P1-derived artificial chromosomes (PACs); and bacteriophages such as λ bacteriophages or M13 bacteriophages and animal viruses. Animal viruses that can be used as vectors include, but are not limited to, retroviruses (including lentiviruses), adenoviruses, adeno-associated viruses, herpes viruses (e.g., herpes simplex viruses), poxviruses, baculoviruses, papillomaviruses, and papovaviruses (e.g., SV40). A vector may comprise a variety of elements for controlling expression, including, but not limited to, promoter sequences, transcription initiation sequences, enhancer sequences, selection elements, and reporter genes. In addition, the vector may also comprise a replication origin.

As used herein, the term "host cell" refers to a cell that can be used for introducing a vector, including but not limited to prokaryotic cells such as Escherichia coli or Bacillus subtilis, fungal cells such as yeast cells or Aspergillus, insect cells such as S2 Drosophila cells or Sf9, or animal cells such as fibroblasts, CHO cells, COS cells, NSO cells, HeLa cells, BHK cells, HEK 293 cells or human cells.

As used herein, the term "conservative substitution" means an amino acid substitution that does not adversely affect or alter the desired properties of the protein/polypeptide comprising the amino acid sequence. For example, conservative substitutions can be introduced by standard technologies known in the art, such as site-directed mutagenesis and PCR-mediated mutagenesis. Conservative amino acid substitutions include substitution of an amino acid residue with an amino acid residue having a similar side chain, such as a residue that is physically or functionally similar to the corresponding amino acid residue (e.g., having similar size, shape, charge and chemical properties, including the ability to form covalent bonds or hydrogen bonds). Families of amino acid residues having similar side chains have been defined in the art. These families include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine), and aromatic side chains (e.g., tyrosine, phenylalanine, tryptophan, and histidine). Thus, it is preferred to replace a corresponding amino acid residue with another amino acid residue from the same side chain family. Methods for identifying conservative amino acid substitutions are well known in the art (see, e.g. Brummell et al., Biochem. 32:1180-1187 (1993); Kobayashi et al. Protein Eng. 12(10):879-884 (1999); and Burks et al. Proc. Natl Acad. Set USA 94:412-417 (1997), which is incorporated herein by reference).

Twenty conventional amino acids mentioned herein are written in accordance with conventional usage. See, e.g. Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. Moreover, in the present invention, amino acids are generally represented by single-letter and three-letter abbreviations well known in the art. For example, alanine can be represented by A or Ala.

As used herein, the term "pharmaceutically acceptable carrier and/or excipient" refers to carriers and/or excipients that are pharmacologically and/or physiologically compatible with the subject and the active ingredient and that are well known in the art, including but not limited to: pH adjusters, surfactants, adjuvants, ionic strength enhancers, diluents, agents for maintaining osmotic pressure, agents for delaying absorption, preservatives. For example, pH adjusting agents include, but are not limited to, phosphate buffers. Surfactants include, but are not limited to, cationic, anionic or nonionic surfactants, such as Tween-80. Ionic strength enhancers include, but are not limited to, sodium chloride. Reagents for maintaining osmotic pressure include, but are not limited to, sugars, NaCl, and analogs thereof. Reagents for delaying absorption include, but are not limited to, monostearates and gelatin. Diluents include, but are not limited to, water, aqueous buffers (e.g., buffered saline), alcohols, polyols (e.g., glycerol), etc. Preservatives include, but are not limited to, various antibacterial reagents and antifungal reagents such as thimerosal, 2-phenoxyethanol, parabens, chlorobutanol, phenol, sorbic acid, etc. Stabilizers have the meanings generally understood by a person of skill in the art, which are capable of stabilizing the desired activity of active ingredients in drugs, and include, but are not limited to, sodium glutamate, gelatin, SPGA, saccharides (e.g., sorbitol, mannitol, starch, sucrose, lactose, dextran, or glucose), amino acids (e.g., glutamic acid and glycine), proteins (e.g., dried whey, albumin or casein), or degradation products thereof (e.g., lactalbumin hydrolysate), etc. In some exemplary embodiments, the pharmaceutically acceptable carrier or excipient includes sterile injectable liquid (e.g., an aqueous or non-aqueous suspension or solution). In some exemplary embodiments, such sterile injectable liquid is selected from water for injection (WFI), bacteriostatic water for injection (BWFI), a sodium chloride solution (e.g., 0.9% NaCl), a glucose solution (e.g., 5% glucose), a solution comprising a surfactant (e.g., 0.01% polysorbate 20), a pH buffer (e.g., phosphate buffer), a Ringer's solution, and any combination thereof.

As used herein, the term "prevention" refers to a method implemented for preventing or delaying the occurrence of a disease or disorder or symptom in a subject. As used herein, the term "treatment" refers to a method implemented to enable a subject who exhibits symptoms of a disease or is diagnosed with a disease to achieve beneficial or desired clinical outcomes. For purposes of the present invention, beneficial or desired clinical results include, but are not limited to, alleviation of symptoms, reduction in the extent of diseases, stabilization (i.e., no longer worsening) of disease states, delay or slowing of disease progression, amelioration or palliation of disease states, and relief of symptoms (regardless partial or complete), regardless detectable or undetectable. In addition, the "treatment" may also mean prolonging the survival time as compared to the expected survival time (e.g., survival time without treatment).

As used herein, the term "subject" refers to a mammal, such as a human or a non-human primate (e.g., cynomolgus monkey). In some embodiments, the subject suffers from autoimmune diseases, allergic diseases, graft-versus-host disease, transplant rejection, or lymphoma (such as T-cell lymphoma), or has a risk of developing these diseases.

As used herein, the term "effective amount" refers to an amount sufficient to achieve or at least partially achieve desired effects. For example, an effective amount for preventing a disease (e.g., autoimmune diseases, allergic diseases, graft-versus-host disease) refers to an amount sufficient to prevent, inhibit or delay the occurrence of the disease; and an effective amount for treating a disease refers to an amount sufficient to cure or at least partially inhibit the disease and complications thereof in a patient suffering from the disease. Assay of such effective amounts is completely within the capability of those skilled in the art. For example, amounts effective for therapeutic use will depend on the severity of the disease to be treated, the overall state of the immune system of the patient, the general conditions of the patient such as age, weight and sex, the manner of administration of a drug, other therapies administered simultaneously, and the like.

### Beneficial effects of the present invention

The anti-PD-1 monoclonal antibody provided by the present invention not only does not block the binding of PD-1 to PD-L1/PD-L2, but can also enhance the binding of PD-1 to PD-L1/PD-L2, activate the PD-1 signaling pathway, and inhibit T cell activity, thereby suppressing the immune response or inflammatory reaction. Therefore, the anti-PD-1 monoclonal antibody of the present invention has important clinical significance for the treatment of inflammation, allergies and autoimmune-related diseases. Furthermore, the antibody of the present invention has cross-species binding activity and activation activity against human and monkey PD-1, which is beneficial for conducting preclinical evaluation studies in animal models.

The embodiments of the present invention will be described in detail below in conjunction with the accompanying drawings and examples, but a person of skill in the art will understand that the following accompanying drawings and examples are only used for illustrating the present invention rather than limiting the scope of the present invention. Various purposes and advantages of the present invention will become implementable to those skilled in the art according to the accompanying drawings and the following detailed descriptions of the preferred embodiments.

### BRIEF DESCRIPTION OF THE DRAWINGS

Fig. 1: Assay results of anti-PD-1 chimeric antibody inhibiting the activation of Jurkat-PD1-NFAT reporter cells.
Fig. 2: Assay results of the binding of anti-PD-1 humanized antibodies to CHOS-hPD-1.
Fig. 3: Assay results of the binding of anti-PD-1 humanized antibodies to CHOS-cPD-1.
Fig. 4: Assay results of anti-PD-1 humanized antibodies inhibiting the activation of Jurkat-PD1-NFAT reporter cells.
Fig. 5: Assay results of anti-PD-1 humanized antibodies inhibiting the IFN-γ secretion activity of primary T cells.
Fig. 6: Assay results of the ADCC activity of anti-PD-1 humanized antibodies.
Fig. 7: Assay results of anti-PD-1 humanized antibody blocking or enhancing the binding of hPD-L1 to CHOS-hPD-1.
Fig. 8: Assay results of anti-PD-1 humanized antibody blocking or enhancing the binding of hPD-L2 to CHOS-hPD-1.
Fig. 9: Assay results of the efficacy of anti-PD-1 humanized antibodies in a mouse GVHD model.
Fig. 10: Assay results of the pharmacokinetic of anti-PD-1 humanized antibodies in human FcRn-transgenic mice.
Fig. 11: Assay results of the efficacy of anti-PD-1 humanized antibodies in a mouse SLE model.
Fig. 12: Assay results of the efficacy of anti-PD-1 humanized antibodies in a mouse TNBS-IBD model.

### Sequence information

**Table 1: Information about the sequences involved in the present invention is described in the table below:**

| SEQ ID NO: | Sequence description |
|---|---|
| 1 | B1-1B5-H1L1/1B5-zu1-hIgG1/1B5-zu1-hIgG1 DD heavy chain variable region CDR1 amino acid sequence (NYPIE) |
| 2 | B1-1B5-H1L1/1B5-zu1-hIgG1/1B5-zu1-hIgG1 DD heavy chain variable region CDR2 amino acid sequence (NFHPYNDDTKYNEKFKG) |
| 3 | B1-1B5-H1L1/1B5-zu1-hIgG1/1B5-zu1-hIgG1 DD heavy chain variable region CDR3 amino acid sequence (EDYGTYGGFAY) |
| 4 | B1-1B5-H1L1/1B5-zu1-hIgG1/1B5-zu1-hIgG1 DD light chain variable region CDR1 amino acid sequence (RASSSVSSSYLL) |
| 5 | B1-1B5-H1L1/1B5-zu1-hIgG1/1B5-zu1-hIgG1 DD light chain variable region CDR2 amino acid sequence (STSNLAS) |
| 6 | B1-1B5-H1L1/1B5-zu1-hIgG1/1B5-zu1-hIgG1 DD light chain variable region CDR3 amino acid sequence (QQYSGYPLT) |
| 7 | B1-1B5-H1L1 heavy chain variable region amino acid sequence |
| 8 | B1-1B5-H1L1 light chain variable region amino acid sequence |
| 9 | B1-1B5-H1L1 heavy chain variable region nucleotide sequence |
| 10 | B1-1B5-H1L1 light chain variable region nucleotide sequence |
| 11 | B1-1B5-H1L1 heavy chain amino acid sequence |
| 12 | B1-1B5-H1L1 light chain amino acid sequence |
| 13 | B1-1B5-H1L1 heavy chain nucleotide sequence |
| 14 | B1-1B5-H1L1 light chain nucleotide sequence |
| 15 | 1B5-zu1-hIgG1/1B5-zu1-hIgG1 DD heavy chain variable region amino acid sequence |
| 16 | 1B5-zu1-hIgG1/1B5-zu1-hIgG1 DD light chain variable region amino acid sequence |
| 17 | 1B5-zu1-hIgG1/1B5-zu1-hIgG1 DD heavy chain variable region nucleotide sequence |
| 18 | 1B5-zu1-hIgG1/1B5-zu1-hIgG1 DD light chain variable region nucleotide sequence |
| 19 | 1B5-zu1-hIgG1 heavy chain amino acid sequence |
| 20 | 1B5-zu1-hIgG1/1B5-zu1-hIgG1 DD light chain amino acid sequence |
| 21 | 1B5-zu1-hIgG1 heavy chain nucleotide sequence |
| 22 | 1B5-zu1-hIgG1/1B5-zu1-hIgG1 DD light chain nucleotide sequence |
| 23 | 1B5-zu1-hIgG1 DD heavy chain amino acid sequence |
| 24 | 1B5-zu1-hIgG1 DD heavy chain nucleotide sequence |
| 25 | B1-1C4-H1L1/1C4-zu1-hIgG1/1C4-zu1-hIgG1 DD heavy chain variable region CDR1 amino acid sequence (SYWIT) |
| 26 | B1-1C4-H1L1/1C4-zu1-hIgG1/1C4-zu1-hIgG1 DD heavy chain variable region CDR2 amino acid sequence (RIAPGSGSIYYDEMFKD) |
| 27 | B1-1C4-H1L1/1C4-zu1-hIgG1/1C4-zu1-hIgG1 DD heavy chain variable region CDR3 amino acid sequence (LGNYGWTMDY) |
| 28 | B1-1C4-H1L1/1C4-zu1-hIgG1/1C4-zu1-hIgG1 DD light chain variable region CDR1 amino acid sequence (KASQSVTNNVA) |
| 29 | B1-1C4-H1L1/1C4-zu1-hIgG1/1C4-zu1-hIgG1 DD light chain variable region CDR2 amino acid sequence (YVSNRYT) |
| 30 | B1-1C4-H1L1/1C4-zu1-hIgG1/1C4-zu1-hIgG1 DD light chain variable region CDR3 amino acid sequence (QQDYSSWT) |
| 31 | B1-1C4-H1L1 heavy chain variable region amino acid sequence |
| 32 | B1-1C4-H1L1 light chain variable region amino acid sequence |
| 33 | B1-1C4-H1L1 heavy chain variable region nucleotide sequence |
| 34 | B1-1C4-H1L1 light chain variable region nucleotide sequence |
| 35 | B1-1C4-H1L1 heavy chain amino acid sequence |
| 36 | B1-1C4-H1L1 light chain amino acid sequence |
| 37 | B1-1C4-H1L1 heavy chain nucleotide sequence |
| 38 | B1-1C4-H1L1 light chain nucleotide sequence |
| 39 | 1C4-zu1-hIgG1/1C4-zu1-hIgG1 DD heavy chain variable region amino acid sequence |
| 40 | 1C4-zu1-hIgG1/1C4-zu1-hIgG1 DD light chain variable region amino acid sequence |
| 41 | 1C4-zu1-hIgG1/1C4-zu1-hIgG1 DD heavy chain variable region nucleotide sequence |
| 42 | 1C4-zu1-hIgG1/1C4-zu1-hIgG1 DD light chain variable region nucleotide sequence |
| 43 | 1C4-zu1-hIgG1 heavy chain amino acid sequence |
| 44 | 1C4-zu1-hIgG1/1C4-zu1-hIgG1 DD light chain amino acid sequence |
| 45 | 1C4-zu1-hIgG1 heavy chain nucleotide sequence |
| 46 | 1C4-zu1-hIgG1/1C4-zu1-hIgG1 DD light chain nucleotide sequence |
| 47 | 1C4-zu1-hIgG1 DD heavy chain amino acid sequence |
| 48 | 1C4-zu1-hIgG1 DD heavy chain nucleotide sequence |
| 49 | Full-length hPD-1 amino acid sequence |
| 50 | hPD-1 ECD amino acid sequence |
| 51 | hIgG1 heavy chain constant region amino acid sequence |
| 52 | hIgG1(DD) heavy chain constant region amino acid sequence |
| 53 | Human kappa light chain constant region sequence |
| 54 | cPD-1 amino acid sequence |
| 55 | cPD-1 ECD amino acid sequence |

### Examples

The present invention will now be described in the following non-limiting examples.

Those skilled in the art will appreciate that the Examples describe the present invention by way of examples and are not intended to limit the scope of protection claimed for the present application. The experimental methods in the examples are all conventional methods, unless otherwise specified. Examples, in which no specific conditions are specified, are carried out according to conventional conditions or conditions recommended by the manufacturers. The reagents and instruments used without indicating the manufacturer are all conventional products that can be purchased commercially.

### Example 1: Preparation of antigen proteins

### 1.1 Transient transfection and expression of antigens

Transient transfection and expression of hPD-1 ECD mIgG1Fc and cPD-1 ECD mIgG1Fc was performed using FreeStyle^{TM}293F cells (Gibco) in Expi293 Expression Medium (Gibco). 24 h before transfection, 100 mL of Expi293F cells at 2.5-3×10⁶ cells/mL were seeded into a 500 mL cell culture flask, and the cell culture flask was placed in a shaker for culture at 37°C, 8% CO₂, and 120 rpm. Before transfection, the cell density was measured. When it reached 4.5-5.5×10⁶ cells/mL, the cell density was adjusted to 3×10⁶ cells/mL and transfection was performed. For transfection, 320 µL of ExpiFectamine^{TM} 293 Reagent (Gibco) was first added to 5.68 mL of Opti-MEM (Life), mixed well, and incubated at room temperature for 5 min; at the same time, 100 µg each of the expression plasmids of hPD-1 ECD mIgG1Fc and cPD-1 ECD mIgG1Fc was diluted to 6 mL using Opti-MEM. The diluted transfection reagent and plasmid were thoroughly mixed and incubated at room temperature for 10-20 min, and then all the mixture was added to the cells and mixed well. The culture flask was placed in a shaker for culture at 37°C, 8% CO₂, and 120 rpm for 5 days. 18-22 h after cell transfection, 600 µL of ExpiFectamin 293 enhancer 1 and 6 mL of enhancer 2 (Gibco) were added.

### 1.2 Antigen purification

The cell culture was centrifuged at 2000g for 20 min, the supernatant was collected and filtered through a 0.22-µm filter membrane. After PrismA (Cytiva) affinity chromatography (protein purification liquid chromatography system: Cytiva, AKTA avant 25), elution was performed with 20 mM citrate-sodium citrate (pH 3.2), followed by pH adjustment to neutral with 1 M Tris base. The purity of proteins in the purified sample was determined by SDS-PAGE using a 4-20% gradient gel.

### Example 2: Generation of anti-PD-1 monoclonal antibody based on single B cell cloning technology

### 2.1 Mouse immunization

Female BALB/c mice aged 6-8 weeks (SPF (Beijing) Biotechnology Co., Ltd., Animal License No.: SCXK (Jing) 2019-0010) were housed in an SPF-grade laboratory with a 12/12-hour light/dark cycle, at a temperature of 20-26°C and a humidity of 40-70%. Before immunization, 10 µg of hPD-1 ECD mIgG1Fc or cPD-1 ECD mIgG1Fc protein was mixed with an adjuvant at a volume ratio of 1:1. Ten mice were subjected to plantar immunization. The first immunization was performed using Freund's complete adjuvant, followed by a second immunization two days later using Freund's incomplete adjuvant. The volume injected at each injection site was 25 µL. Three weeks after immunization, blood was collected from the orbital cavity of mice, and serum titer was detected by indirect ELISA. Mice with a titer of 1:200,000 or higher were given booster immunization.

### 2.2 Plasma cell enrichment

Three to five days after the mice received booster immunization, they were euthanized by cervical dislocation and then immersed in 75% alcohol for 5 min. The abdominal skin and leg muscles were incised. At the highly vascularized regions within the abdominal skin and leg muscles, the corresponding lymph nodes were lifted with fine forceps, excised, trimmed of non-lymphoid tissues, and transferred into Gibco RPMI1640 medium. The skin and peritoneum at the junction of the left abdomen and chest of the mouse were cut open to expose the spleen. The spleen was lifted with fine forceps, excised, trimmed of non-splenic tissues, and transferred into Gibco RPMI1640 medium.

The leg muscles of the mice were carefully dissected; the femurs and tibias were excised separately, the cartilages at both ends were trimmed off to expose the red medullary cavities, and the residual muscles on the bones were completely removed with lint-free cloths. A 1 mL sterile syringe was taken, 1 mL of RPMI1640 medium was drawn up, and the sterile needle was gently inserted into the medullary cavity to flush the cavity and harvest bone marrow. A 40 µm filter mesh was placed inside a 50 mL centrifuge tube; bone marrow fluid was drawn up with a pipette and slowly passed through the filter mesh into the centrifuge tube to remove residues. The centrifuge tube was placed in a centrifuge and centrifuged for 10 min (400g, 4°C). The supernatant was removed, EasySep^{™} Buffer (STEMCELL Technologies) was added, the mixture was mixed thoroughly, and then subjected to bone marrow cell counting.

The spleens and lymph nodes were ground with a syringe plunger; after grinding, the cells were filtered through a 40 µm filter mesh, and the cell suspensions were transferred separately into sterile centrifuge tubes. Following centrifugation, the supernatants were discarded, and the lymphocytes were resuspended in 5 mL of Gibco RPMI1640 medium for subsequent use. A 10 µL aliquot of the cell suspension was taken for counting the splenic and lymph node cells. Plasma cells were then enriched using the EasySep^{™} Mouse CD138 Positive Selection kit (STEMCELL Technologies).

### 2.3 Screening of plasma cells that specifically bind to PD-1 antigen using the Beacon single-cell photoconductive system

Biotin-labeled hPD-1 or cPD-1 protein was coated with assay beads (Berkeley Lights), and then the coated antigen beads were washed with PBS for later use. After adjusting the density of the enriched plasma cells to 7×10⁶/mL, the cells were loaded into the NanoPen of the OptoSelect 14K Chip (Berkeley Lights). The hPD-1 or cPD-1 proteincoated assay beads and the PE Goat Anti-Mouse IgG secondary antibody (Jackson) at a concentration of 5 µg/mL were introduced into the chip's Channel. The exposure time for the PE fluorescence channel was set to 1000ms, with a total of 7 cycles, each cycle lasting 6 min. After the experiment was completed, the plasma cells that tested positive for both hPD-1 and cPD-1 proteins were exported into a 96-well plate containing lysis buffer and mineral oil.

### 2.4 Plasma cell sequencing and expression vector construction

cDNA synthesis was performed using the Opto Plasma B Discovery cDNA Synthesis Kit (Berkeley Lights). Then, the IgG heavy chain variable region gene and the Kappa chain variable region gene were amplified using the Opto Plasma B Discovery Sanger Prep Kit (Berkeley Lights). The amplified heavy chain variable region and light chain variable region fragments were subjected to homologous recombination, transformation, and sequencing with the vector pcDNA3.4(EBV)-hIgG1CH-S1 containing the human IgG1 heavy chain constant region sequence (SEQ ID NO: 51) and the vector pcDNA3.4(EBV)-hIgKCL-S1 containing the human IgK light chain constant region sequence (SEQ ID NO: 53), respectively.

Two anti-PD-1 chimeric antibodies, B1-1B5-H1L1 and B1-1C4-H1L1, were finally obtained, and their sequence information is shown in the table below.

**Table 2: Sequence information of anti-PD-1 chimeric antibodies**

| | SEQ ID NO: | | | | | | | | | |
|---|---|---|---|---|---|---|---|---|---|---|
| Antibody | VH | | | VH | VL | | | VL | CH | CL |
| | CDR1 | CDR2 | CDR3 | | CDR1 | CDR2 | CDR3 | | | |
| B1-1B5-H1L1 | 1 | 2 | 3 | 7 | 4 | 5 | 6 | 8 | 51 | 53 |
| B1-1C4-H1L1 | 25 | 26 | 27 | 31 | 28 | 29 | 30 | 32 | 51 | 53 |

### Example 3: Preparation of recombinant anti-PD-1 chimeric antibodies

### 3.1 Transient transfection and expression of antibodies

24 h before transfection, 100 mL of Expi293F cells at 2.5-3×10⁶ cells/mL were seeded into a 500 mL cell culture flask, and the cell culture flask was placed in a shaker for culture at 37°C, 8% CO₂, and 120 rpm. Before transfection, the cell density was measured. When it reached 4.5-5.5×10⁶ cells/mL, the cell density was adjusted to 3×10⁶ cells/mL and transfection was performed. In a 96-well PCR plate, the plasmid was diluted to 20 ng/µL using Opti-MEM (Gibco), 40 µL of the diluted plasmid was slowly added to a U-bottom 96-well plate pre-filled with 50 µL/well of Opti-MEM (total plasmid amount: 0.8 µg, 0.4 µg each of light and heavy chain plasmids). For transfection, 2.5 µL of ExpiFectamine^{™} 293 Reagent (Gibco) was added to 50 µL of Opti-MEM and mixed well. The mixture was then incubated at room temperature for 5 min. The above diluted transfection reagent and plasmid were mixed thoroughly and incubated at room temperature for 10-20 min; 0.8 mL of cell suspension was added to a 96-well cell culture plate and 0.8 mL of PBS was added to the remaining wells. The prepared transfection system solution was slowly added to the cells, and the cell culture plate was placed in a cell shaker at 37°C, 8% CO₂, and 80-85% humidity, and culture was performed with shaking at 900 rpm for 5 days. 18-22 h after cell transfection, 5 µL of ExpiFectamin 293 enhancer 1 and 50 µL of ExpiFectamin 293 enhancer 2 (Gibco) were added.

### 3.2 Antibody purification

The supernatant was collected into a 96-well plate, PrismA affinity chromatography medium (Cytiva) was added to the supernatant, and incubation was performed for 2 h for binding. The system was equilibrated with 20mM PB-150mM NaCl, eluted with 20mM citric acid-sodium citrate (pH 3.2), and then adjusted to make the pH neutral with 1M Tris base. The purity of antibodies in the purified sample was determined by SDS-PAGE using a 4-20% gradient gel.

### Example 4: Assay of binding activity between anti-PD-1 chimeric antibodies and PD-1 overexpressing cell lines

### 4.1 Preparation of PD-1 overexpressing cell lines

The Homo sapiens PD-1 protein encoding gene (gene ID: NM_005018.3) was ligated into the pLVX-hPD-1-IRES-Neo plasmid to construct a lentiviral plasmid for the hPD-1 protein. The Mus musculus PD-1 protein encoding gene (gene ID: NM_008798.3) was ligated into the pLVX-mPD-1-IRES-Hyg plasmid to construct a lentiviral plasmid for the mPD-1 protein. The Macaca fascicularis PD-1 protein encoding gene (gene ID: EF443145.1) was ligated into the pLVX-cPD-1-IRES-Hyg plasmid to construct a lentiviral plasmid for the cPD-1 protein.

Lentiviral packaging was performed using a three-plasmid system (enveloping plasmid pCMV-VSVG, packaging plasmid pCMV-dr8.91, and target plasmid pLvx backbone plasmid), followed by concentration to obtain a lentivirus solution finally. CHOS cells were infected using the lentivirus solution. After infection, the infected cells were subjected to selection pressure with 500 µg/mL GENETICIN (Gibco) or 500 µg/mL Hygromycin B (Invitrogen) to obtain resistant Pool. The expression of hPD-1, mPD-1, and cPD-1 proteins on the cell surface was determined using FACS. The identified positive Pool cells were monocloned using the limiting dilution method, and CHOS-hPD-1, CHOS-mPD-1, and CHOS-cPD-1 cell lines overexpressing hPD-1, mPD-1, and cPD-1 proteins were obtained after expansion culture and identification.

### 4.2 Assay of binding activity between antibodies to PD-1 overexpressing cell lines

CHOS-hPD-1, CHOS-mPD-1, CHOS-cPD-1, and CHOS empty cells were resuspended in 1% BSA/1×PBS and then added to 96-well U-shaped plates, with 2×10⁵ cells per well. The antibody was diluted to 10 µg/mL with 1% BSA/1×PBS, 100 µL per well, with 2 replicate wells for each antibody. Meanwhile, NC and Blank were set up, and incubation was performed at 4°C for 30 min. The wells were washed twice with 1% BSA/1×PBS, followed by the addition of Goat Anti-Human IgG (Jackson) secondary antibody diluted at a ratio of 1:200, 100 µL per well, and incubation was performed at 4°C for 30 min. After washing twice with 1% BSA/1×PBS, the cells were resuspended in 50 µL of 1% BSA/1×PBS. The cells were analyzed using a flow cytometer (BD Canto II). The results were analyzed using FlowJo software, and the mean fluorescence intensity (MFI) was calculated. The antibodies exhibiting an MFI value of binding to CHOS-hPD-1 or CHOS-cPD-1 that is more than 10 times that of the NC were selected for subsequent experiments.

### Example 5: Assay of the activity of anti-PD-1 chimeric antibodies in inhibiting Jurkat-PD1-NFAT reporter cell activation

Jurkat-PD1-NFAT cells (purchased from the National Institutes for Food and Drug Control) were adjusted to a density of 2 ×10⁶/mL using RPMI-1640 experimental medium (Gibco, C11875500BT) supplemented with 1% FBS (Gibco, 10091-148), and then seeded into a 96-well white plate (CORNING, 3917) at 25 µL/well, that is, 5×10⁴ cells/well. The antibody to be tested was diluted with the experimental medium and added to a 96-well plate at 25 µL/well, with a highest concentration of 75 nM, 4-fold serial dilution, and 9 gradients. THP-1 cells (purchased from BNCC) were adjusted to a density of 1.6×10⁶/mL using the experimental medium, and then seeded into a 96-well plate at 25 µL/well, that is, 4×10⁴ cells/well. The CD3 antibody (homemade) was diluted with the experimental medium and added to the corresponding assay wells at 25 µL/well, resulting in a final concentration of 0.01 nM. The total experimental system was 100 µL/well, and was incubated at a 37°C, 5% CO₂ incubator for 6-24 h. Bright-Lite (Vazyme, DD1204-02) was added at 100 µL/well, followed by incubation for 3 min at room temperature in the dark; the luminescence value was measured using the SpectraMax iD5 Multimode Microplate Reader (Molecular Devices). Formula for calculating reporter gene inhibition rate: Reporter gene inhibition (%) = 100%×[1-(RLUsample-RLUcell)/(RLUcontrol-RLUcell)].

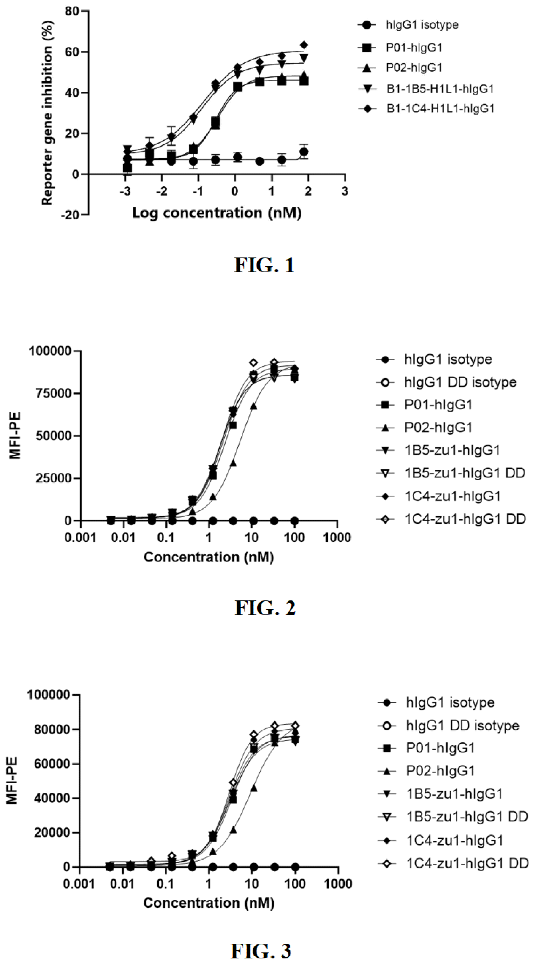

The results are shown in Fig. 1 and Table 3. The anti-PD-1 chimeric antibodies B1-1B5-H1L1 and B1-1C4-H1L1 showed dose-dependent inhibition of Jurkat-PD1-NFAT reporter cell activation, and their inhibitory activity was superior to that of the positive controls P01-hIgG1 (refer to patent US20190270818A1, heavy chain sequence: SEQ ID NO: 1; light chain sequence: SEQ ID NO: 2) and P02-hIgG1 (refer to patent WO2020247648A2, heavy chain sequence: SEQ ID NO: 51; light chain sequence: SEQ ID NO: 52), as evidenced by an EC50 that was 2-3 times lower than that of the positive controls P01-hIgG1 and P02-hIgG1. The heavy chain constant region sequences of the two positive control antibodies are identical to that of SEQ ID NO: 51 in the present application, and the hIgG1 isotype is an unrelated negative control antibody.

**Table 3: Results of the assay for inhibition of Jurkat-PD1-NFAT reporter cell activation by anti-PD-1 chimeric antibodies**

| Antibody name | EC50 (nM) |
|---|---|
| hIgG1 isotype | NA |
| P01-hIgG1 | 0.30 |
| P02-hIgG1 | 0.33 |
| B1-1B5-H1L1 | 0.12 |
| B1-1C4-H1L1 | 0.14 |

### Example 6: Humanization of anti-PD-1 antibodies

The murine antibodies B1-1B5-H1L1 and B1-1C4-H1L1 obtained via screening were aligned and analyzed against a known human antibody database to identify the germline genes with the highest homology, the framework regions of which were selected for use (with the CDRs and framework regions defined according to the Kabat numbering system). Subsequently, through computer-aided predictive simulation, the CDRs were replaced with the corresponding CDR sequences derived from the parental antibodies. Furthermore, back mutations were introduced to retain the murine amino acid residues in the framework regions of the parental antibodies that exert critical effects on antigen binding. Finally, the variable regions 1B5-zu1 (heavy chain variable region sequence: SEQ ID NO: 15; light chain variable region sequence: SEQ ID NO: 16) and 1C4-zu1 (heavy chain variable region sequence: SEQ ID NO: 39; light chain variable region sequence: SEQ ID NO: 40) of the humanized antibodies of B1-1B5-H1L1 and B1-1C4-H1L1 were generated respectively.

### Example 7: Preparation of anti-PD-1 humanized antibodies

### 7.1 Transient transfection and expression of humanized antibodies

The heavy chain variable region and light chain variable region of the humanized antibody in Example 6 were ligated to the heavy chain constant region and light chain constant region, respectively, to obtain 4 heavy chain expression vectors and 4 light chain expression vectors. These vectors were then combined and transfected for antibody expression. The amino acid sequences and nucleotide sequences of the antibodies are shown in Table 4. Among them, 1B5-zu1-hIgG1 DD and 1C4-zu1-hIgG1 DD are Fc region modified mutants of 1B5-zu1-hIgG1 and 1C4-zu1-hIgG1, respectively. DD represents the S239D/H268D mutant (EU numbering system), and the sequence of this Fc variant is set forth in SEQ ID NO: 52.

**Table 4: Amino acid and nucleotide sequences of anti-PD-1 humanized antibodies**

| Antibody name | Heavy chain amino acid Sequence | Light chain amino acid Sequence | Heavy chain nucleotide Sequence | Light chain nucleotide Sequence |
|---|---|---|---|---|
| 1B5-zu1-hIgG1 | SEQ ID NO: 19 | SEQ ID NO: 20 | SEQ ID NO: 21 | SEQ ID NO: 22 |
| 1B5-zu1-hIgG1 DD | SEQ ID NO: 23 | SEQ ID NO: 20 | SEQ ID NO: 24 | SEQ ID NO: 22 |
| 1C4-zu1-hIgG1 | SEQ ID NO: 43 | SEQ ID NO: 44 | SEQ ID NO: 45 | SEQ ID NO: 46 |
| 1C4-zu1-hIgG1 DD | SEQ ID NO: 47 | SEQ ID NO: 44 | SEQ ID NO: 48 | SEQ ID NO: 46 |

24h before transfection, 100 mL of ExpiCHO-S at 3-4×10⁶ cells/mL were seeded into a 500 mL cell culture flask, and the cell culture flask was placed in a shaker for culture at 37°C, 8% CO₂, and 120 rpm. Before transfection, the cell density was measured. When it reached 6-8×10⁶ cells/mL, the cell density was adjusted to 6×10⁶ cells/mL and transfection was performed. During transfection, 100 µg of each of the expression plasmids used for 1B5-zu1-hIgG1, 1B5-zu1-hIgG1 DD, 1C4-zu1-hIgG1 and 1C4-zu1-hIgG1 DD molecules (light chain to heavy chain expression plasmid ratio of 1:1) were diluted to 4 mL with Opti-PRO SFM. Then, 320 µL of ExpiFectamine CHO Reagent was added to 3.68 mL of OptiPRO SFM and mixed well. The mixture was then incubated at room temperature for 2 min. The diluted transfection reagent and plasmid were thoroughly mixed and incubated at room temperature for 2-5 min, and then all the mixture was added to the cells and mixed well. The culture flask was placed in a shaker for culture at 37°C, 8% CO₂, and 120 rpm. 18-22 h after cell transfection, 600 µl of ExpiFectamine CHO Enhancer and 16 mL of ExpiCHO Feed were added to the culture system, and the culture conditions were adjusted to 32°C, 5% CO₂, and 120 rpm. On the fifth day after transfection, 16 mL of ExpiCHO Feed was added, followed by continued culture for 12-14 days.

### 7.2 Purification of humanized antibodies

The cell culture was centrifuged at 2000g for 20 min, the supernatant was collected and filtered through a 0.22-µm filter membrane. After PrismA (Cytiva) affinity chromatography (protein purification liquid chromatography system: Cytiva, AKTA avant 25), elution was performed with 20 mM citrate-sodium citrate (pH 3.2), followed by pH adjustment to neutral with 1 M Tris base. The purity of antibodies in the purified sample was determined by SDS-PAGE using a 4-20% gradient gel.

### Example 8: Assay of affinity between anti-PD-1 humanized antibodies and PD-1 antigens

Using a Sensor Chip Protein A biosensor chip (Cytiva, 29-1275-56), 1B5-zu1-hIgG1, 1B5-zu1-hIgG1 DD, 1C4-zu1-hIgG1, and 1C4-zu1-hIgG1 DD were immobilized on the chip as ligands. Different concentrations of antigens hPD-1 ECD-Avi-His and cPD-1 ECD-Avi-His were flowed through the chip at a flow rate of 30 µL/min to facilitate antigen-antibody binding. The association time was 180 s and the dissociation time was 180 s. Kinetic fitting was performed using Biacore 8K Evaluation (Cytiva), and the affinity constants obtained are shown in Tables 5 and 6 below. 1B5-zu1-hIgG1 and 1B5-zu1-hIgG1 DD have comparable affinity to hPD-1 ECD-Avi-His; 1C4-zu1-hIgG1 and 1C4-zu1-hIgG1 DD have comparable affinity to hPD-1 ECD-Avi-His. 1B5-zu1-hIgG1 and 1B5-zu1-hIgG1 DD have comparable affinity to cPD-1 ECD-Avi-His; 1C4-zu1-hIgG1 and 1C4-zu1-hIgG1 DD have comparable affinity to cPD-1 ECD-Avi-His.

**Table 5: Assay results of affinity between anti-PD-1 humanized antibodies and hPD-1**

| Antibody name | Ka (1/Ms) | Kd (1/s) | KD (M) |
|---|---|---|---|
| 1B5-zu1-hIgG1 | 2.04E+05 | 2.40E-03 | 1.18E-08 |
| 1B5-zu1-hIgG1 DD | 2.09E+05 | 2.31E-03 | 1.11E-08 |
| 1C4-zu1-hIgG1 | 2.36E+05 | 1.95E-03 | 8.26E-09 |
| 1C4-zu1-hIgG1 DD | 2.40E+05 | 1.94E-03 | 8.05E-09 |

**Table 6: Assay results of affinity between anti-PD-1 humanized antibodies and cPD-1**

| Antibody name | Ka (1/Ms) | Kd (1/s) | KD (M) |
|---|---|---|---|
| 1B5-zu1-hIgG1 | 2.19E+05 | 8.41E-03 | 3.85E-08 |
| 1B5-zu1-hIgG1 DD | 2.43E+05 | 8.56E-03 | 3.51E-08 |
| 1C4-zu1-hIgG1 | 2.51E+05 | 3.92E-03 | 1.56E-08 |
| 1C4-zu1-hIgG1 DD | 2.59E+05 | 3.93E-03 | 1.52E-08 |

### Example 9: Assay of affinity between anti-PD-1 humanized antibodies and Fc receptors

An anti-His antibody (Cytiva, 28995056) was conjugated to a CM5 biosensor chip (Cytiva, BR-1005-30). hCD32A (167 His, His Tag, Sino Biological, 10374-H08C1) and hCD32B (ECD, His tag, Sino Biological, 10259-H08C) were used as ligands. Different concentrations of antibodies 1B5-zu1-hIgG1, 1B5-zu1-hIgG1 DD, 1C4-zu1-hIgG1, and 1C4-zu1-hIgG1 DD were flowed through the chip at a flow rate of 30 µL/min to facilitate the binding between the Fc receptor and the antibody. The binding time was 60 s, and the dissociation time was 60 s. Fitting was performed using Biacore 8K Evaluation software (Cytiva), and the affinity constants obtained are shown in Tables 7 and 8 below. hCD32A showed a 3-4 times higher affinity for 1B5-zu1-hIgG1 DD or 1C4-zu1-hIgG1 DD compared to 1B5-zu1-hIgG1 or 1C4-zu1-hIgG1. hCD32B showed a 6-9 times higher affinity for 1B5-zu1-hIgG1 DD or 1C4-zu1-hIgG1 DD compared to 1B5-zu1-hIgG1 or 1C4-zu1-hIgG1. The above results indicate that introducing the DD mutation into the Fc region can further enhance the affinity of the antibody for Fc receptors hCD32A and hCD32B.

**Table 7: Assay results of affinity between anti-PD-1 humanized antibodies and hCD32A**

| Antibody name | KD (M) |
|---|---|
| 1B5-zu1-hIgG1 | 5.43E-06 |
| 1B5-zu1-hIgG1 DD | 1.47E-06 |
| 1C4-zu1-hIgG1 | 4.94E-06 |
| 1C4-zu1-hIgG1 DD | 1.61E-06 |

**Table 8: Assay results of affinity between anti-PD-1 humanized antibodies and hCD32B**

| Antibody name | KD (M) |
|---|---|
| 1B5-zu1-hIgG1 | 1.34E-05 |
| 1B5-zu1-hIgG1 DD | 1.53E-06 |
| 1C4-zu1-hIgG1 | 1.00E-05 |
| 1C4-zu1-hIgG1 DD | 1.68E-06 |

An anti-His antibody was conjugated to a CM5 biosensor chip. Homemade hCD16A(158F)-ECD-C-his and hCD16A(158V)-ECD-C-his were used as ligands. Different concentrations of antibodies 1B5-zu1-hIgG1, 1B5-zu1-hIgG1 DD, 1C4-zu1-hIgG1, and 1C4-zu1-hIgG1 DD were flowed through the chip at a flow rate of 30 µL/min to facilitate the binding between the Fc receptor and the antibody. The binding time was 60&90 s, and the dissociation time was 60&90 s. Kinetic and steady-state fitting was performed using Biacore 8K Evaluation software (Cytiva), and the obtained affinity constants are presented in Tables 9 and 10 below. hCD16A(158F) showed a 3-6 times higher affinity for 1B5-zu1-hIgG1 DD or 1C4-zu1-hIgG1 DD compared to 1B5-zu1-hIgG1 or 1C4-zu1-hIgG1. hCD16A(158V) showed a 3-4 times higher affinity for 1B5-zu1-hIgG1 DD or 1C4-zu1-hIgG1 DD compared to 1B5-zu1-hIgG1 or 1C4-zu1-hIgG1. The above results indicate that introducing the DD mutation into the Fc region can further enhance the affinity of the antibody for Fc receptor hCD16A.

**Table 9: Assay results of affinity between anti-PD-1 humanized antibodies and hCD16A(158F)**

| Antibody name | Ka (1/Ms) | Kd (1/s) | KD (M) |
|---|---|---|---|
| 1B5-zu1-hIgG1 | 9.21E+04 | 3.00E-02 | 3.26E-07 |
| 1B5-zu1-hIgG1 DD | 6.84E+05 | 3.54E-02 | 5.18E-08 |
| 1C4-zu1-hIgG1 | 1.12E+05 | 2.48E-02 | 2.22E-07 |
| 1C4-zu1-hIgG1 DD | 6.74E+05 | 4.23E-02 | 6.27E-08 |

**Table 10: Assay results of affinity between anti-PD-1 humanized antibodies and hCD16A(158V)**

| Antibody name | Ka (1/Ms) | Kd (1/s) | KD (M) |
|---|---|---|---|
| 1B5-zu1-hIgG1 | 9.61E+04 | 7.98E-03 | 8.30E-08 |
| 1B5-zu1-hIgG1 DD | 4.81E+05 | 1.03E-02 | 2.15E-08 |
| 1C4-zu1-hIgG1 | 9.66E+04 | 7.75E-03 | 8.02E-08 |
| 1C4-zu1-hIgG1 DD | 4.45E+05 | 1.13E-02 | 2.54E-08 |

### Example 10: Assay of binding activity between anti-PD-1 humanized antibodies and PD-1 overexpressing cell lines

CHOS-hPD-1 and CHOS-cPD-1 cells were resuspended in 1% BSA/1×PBS and then added to 96-well U-shaped plates, with 2×10⁵ cells per well. Then, the antibodies to be tested with the highest concentration of 100 nM, 3-fold dilution, and 10 concentration gradients were added, respectively, 100 µL per well, and incubation was performed at 4°C for 30 min. The wells were washed twice with 1% BSA/1×PBS, followed by the addition of Goat Anti-Human IgG (Jackson) secondary antibody diluted at a ratio of 1:200, 100 µL per well, and incubation was performed at 4°C for 30 min. After washing twice with 1% BSA/1×PBS, the cells were resuspended in 100 µL of 1% BSA/1×PBS. The cells were analyzed using a flow cytometer (BD Canto II). The results were analyzed using FlowJo software. The mean fluorescence intensity (MFI) value is used to calculate the relative EC50 based on 4-parameter logistic regression.

The results of antibody, CHOS-hPD-1 and CHOS-cPD-1 assays are shown in Fig. 2, Fig. 3 and Table 11, respectively. 1B5-zu1-hIgG1, 1B5-zu1-hIgG1 DD, 1C4-zu1-hIgG1, and 1C4-zu1-hIgG1 DD can bind to CHOS-hPD-1 efficiently, with the binding affinity significantly stronger than that of P02-hIgG1 and slightly superior to that of P01-hIgG1. 1B5-zu1-hIgG1, 1B5-zu1-hIgG1 DD, 1C4-zu1-hIgG1, and 1C4-zu1-hIgG1 DD can bind to CHOS-cPD-1 efficiently, with the binding affinity significantly stronger than that of P02-hIgG1 and slightly superior to that of P01-hIgG1.

**Table 11: Assay results of the binding of anti-PD-1 humanized antibodies to CHOS-hPD-1 and CHOS-cPD-1**

| Antibody name | CHOS-hPD-1 | CHOS-cPD-1 |
|---|---|---|
| | EC50 (nM) | EC50 (nM) |
| hIgG1 isotype | - | - |
| hIgG1 DD isotype | - | - |
| P01-hIgG1 | 15.87 | 22.40 |
| P02-hIgG1 | 35.93 | 62.40 |
| 1B5-zu1-hIgG1 | 11.93 | 20.20 |
| 1B5-zu1-hIgG1 DD | 12.13 | 19.87 |
| 1C4-zu1-hIgG1 | 14.33 | 20.27 |
| 1C4-zu1-hIgG1 DD | 13.67 | 20.07 |

### Example 11: Assay of the activity of anti-PD-1 humanized antibodies in inhibiting Jurkat-PD1-NFAT reporter cell activation

Jurkat-PD1-NFAT cells (purchased from the National Institutes for Food and Drug Control) were adjusted to a density of 2 ×10⁶/mL using RPMI-1640 experimental medium (Gibco, C11875500BT) supplemented with 1% FBS (Gibco, 10091-148), and then seeded into a 96-well white plate (CORNING, 3917) at 25 µL/well, that is, 5×10⁴ cells/well. The antibody to be tested was diluted with the experimental medium and added to a 96-well plate at 25 µL/well, with a highest concentration of 75 nM, 4-fold serial dilution, and 9 gradients. THP-1 cells (purchased from BNCC) were adjusted to a density of 1.6×10⁶/mL using the experimental medium, and then seeded into a 96-well plate at 25 µL/well, that is, 4×10⁴ cells/well. The CD3 antibody (homemade) was diluted with the experimental medium and added to the corresponding assay wells at 25 µL/well, resulting in a final concentration of 0.01 nM. The total experimental system was 100 µL/well, and was incubated at a 37°C, 5% CO₂ incubator for 6-24 h. Bright-Lite (Vazyme, DD1204-02) was added at 100 µL/well, followed by incubation for 3 min at room temperature in the dark; the luminescence value was measured using the SpectraMax iD5 Multimode Microplate Reader (Molecular Devices). Formula for calculating reporter gene inhibition rate: Reporter gene inhibition (%) = 100%×[1-(RLUₛₐₘₚₗₑ-RLU_{cell})/(RLU_{control}-RLU_{cell})].

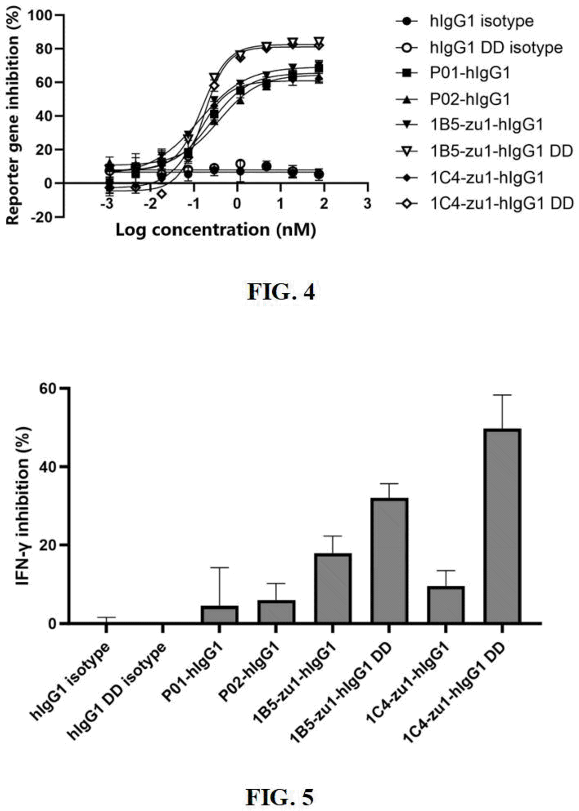

The results are shown in Fig. 4 and Table 12. The humanized antibodies 1B5-zu1-hIgG1 and 1C4-zu1-hIgG1 showed better activity in inhibiting the activation of Jurkat-PD1-NFAT reporter cells than the positive controls P01-hIgG1 and P02-hIgG1, as evidenced by a 2-3 fold reduction in EC50. 1B5-zu1-hIgG1 DD and 1C4-zu1-hIgG1 DD showed superior activity in inhibiting Jurkat-PD1-NFAT reporter cell activation compared to 1B5-zu1-hIgG1 and 1C4-zu1-hIgG1, as evidenced by an approximately 10% increase in maximum inhibition rate.

**Table 12: Assay results of anti-PD-1 humanized antibodies inhibiting the activation of Jurkat-PD1-NFAT reporter cells**

| Antibody name | EC50 (nM) |
|---|---|
| hIgG1 isotype | NA |
| hIgG1 DD isotype | NA |
| P01-hIgG1 | 0.25 |
| P02-hIgG1 | 0.38 |
| 1B5-zu1-hIgG1 | 0.13 |
| 1B5-zu1-hIgG1 DD | 0.15 |
| 1C4-zu1-hIgG1 | 0.12 |
| 1C4-zu1-hIgG1 DD | 0.16 |

### Example 12: Assay for inhibition of IFN-γ secretion activity in primary T cells by anti-PD-1 humanized antibodies

Human CD4⁺ T cells (LePure Biotech) were adjusted to a density of 1×10⁶ cells/mL using RPMI-1640 complete medium containing 10% FBS, and seeded at 2 mL/well into 12-well plates (CORNING, 3513) pre-coated with 3 µg/mL Anti-Human CD3 antibody (clone OKT3, ACRO, CDE-M120a) and stimulated for 3 days. THP-1 cells were treated with mitomycin C (INALCO, 1758-9327) to inhibit cell proliferation: cells at 1×10⁷ cells/mL THP-1 were treated with 10 µg/mL mitomycin C at 37°C for 2 h. After washing THP-1 cells three times with culture medium, they were resuspended in RPMI-1640 experimental medium containing 5% human serum (Hengyuan Biotechnology, HY118-25), and the cell density was adjusted to 5×10⁵ cells/mL, seeded into 96-well U-shaped plates (NEST, 1224B) at 50 µL/well, that is, 2.5×10⁴ cells/well. The antibody to be tested was diluted to 0.25 nM with RPMI-1640 experimental medium containing 5% human serum, and added at 50 µL/well to the corresponding experimental wells. Activated CD4⁺ T cells were collected, diluted with experimental medium to 1×10⁶ cells/mL, and seeded in 96-well U-shaped plates at 50 µL/well, that is, 5×10⁴ cells/well. The CD3 antibody (homemade) was diluted with the experimental medium and added to the corresponding assay wells at 50 µL/well, resulting in a final concentration of 0.04 nM. The total system was 200 µL/well, and was incubated at a 37°C, 5% CO₂ incubator for 18 h. After incubation, the supernatant was collected by centrifugation, and the IFN-γ secretion was detected by ELISA (R&D Systems, SIF50C). The OD value was read using a SpectraMax iD5 multi-functional microplate reader (Molecular Devices) with a detection wavelength of 450 nm and a reference wavelength of 540 nm, and the IFN-γ content was calculated according to the standard curve. Formula for calculating cytokine secretion inhibition rate: inhibition (%) = 100%×[1-(sample-untreated)/(treated-untreated)].

As shown in Fig. 5, 1B5-zu1-hIgG1, 1C4-zu1-hIgG1, 1B5-zu1-hIgG1 DD, and 1C4-zu1-hIgG1 DD all significantly inhibited the secretion of IFN-γ by human CD4⁺ T cells, and their activity was significantly better than that of the positive controls P01-hIgG1 and P02-hIgG1; among them, 1B5-zu1-hIgG1 DD and 1C4-zu1-hIgG1 DD showed better activity in inhibiting the secretion of IFN-γ by human CD4⁺ T cells.

### Example 13: ADCC activity assay for anti-PD-1 humanized antibodies

Human CD56⁺ NK cells (MileCell, Shanghai) were thawed and cultured overnight. The cells were collected the next day and diluted with MEM-α (Gibco, 32561-037) experimental medium containing 1% FBS. The NK effector cells were seeded at 5×10⁴ cells/100µL/well in 96-well plates (CORNING, 3599). The antibodies to be tested were diluted with the experimental medium to a top concentration of 10 nM, followed by 10-fold serial dilution to prepare 8 concentration gradients, and then added to the corresponding experimental wells at 50 µL/well. The target cells CHOS-hPD-1 were diluted with experimental medium and seeded at 1E4 cells/50µL/well in a 96-well plate, corresponding to an effector-to-target ratio (E:T) of 5:1. Meanwhile, the following groups were set up: maximum killing group: 1% Triton + target cells; minimum killing group: target cells; E+T group: effector cells + target cells. The total system was 200 µL/well, the antibodies, target cells and effector cells were mixed, the culture plate was placed in a 37°C, 5% CO₂ incubator for incubation for 24 h. LDH release was detected using the CytoTox 96^{®} NonRadioactive Cytotoxicity Assay Kit (Promega, G1780). The supernatant (50 µL) was collected by centrifugation. Subsequently, LDH substrate was added at 50 µL/well, followed by incubation at room temperature for 20 min in the dark. Then, stop solution was added at 50 µL/well. The OD values of the LDH reaction were measured using the SpectraMax iD5 multimode microplate reader (Molecular Devices) at a test wavelength of 490 nm with a reference wavelength of 620 nm. Calculation formula: %Target cell lysis = 100%×(OD_{Sample} -OD_{E+T})/(OD_{Maximum release}-OD_{Minimum release}).

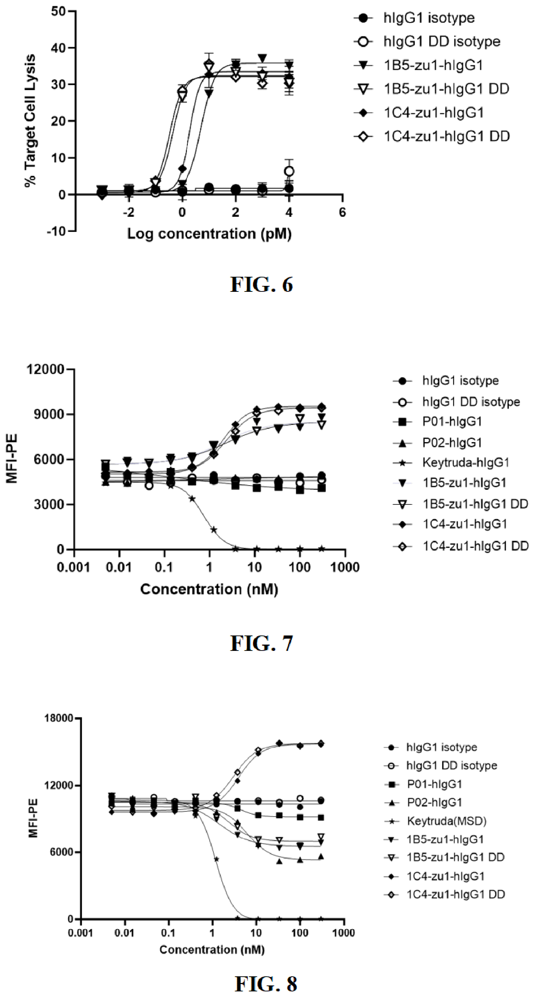

The results are shown in Fig. 6 and Table 13. In the primary human NK cell-mediated ADCC experiment, 1B5-zu1-hIgG1, 1C4-zu1-hIgG1, 1B5-zu1-hIgG1 DD and 1C4-zu1-hIgG1 DD all showed good ADCC activity. Among them, 1B5-zu1-hIgG1 DD and 1C4-zu1-hIgG1 DD showed better ADCC activity, as reflected in a 5-12 fold reduction in EC50.

**Table 13: Assay results of the ADCC activity of anti-PD-1 humanized antibodies**

| Antibody name | EC50 (pM) |
|---|---|
| hIgG1 isotype | NA |
| hIgG1 DD isotype | NA |
| 1B5-zu1-hIgG1 | 5.12 |
| 1B5-zu1-hIgG1 DD | 0.44 |
| 1C4-zu1-hIgG1 | 1.90 |
| 1C4-zu1-hIgG1 DD | 0.33 |

### Example 14: Assay of the activity of anti-PD-1 humanized antibodies in blocking or enhancing the binding of PD-1 to PD-L1 or PD-L2

CHOS-hPD-1 cells were resuspended in 1% BSA/1× PBS and then seeded into a 96-well U-bottom plate at a density of 1×10⁵ cells per well. Then, the antibodies to be tested at the highest concentration of 600 nM, 3-fold dilutions, and 11 concentration gradients were added at 50 µL/well, and the cells were incubated at 4°C for 30 min. 600 nM hPD-L1-hIgG1Fc-Avi(B) or 26.67 nM hPD-L2-hIgG1Fc-Avi(B) was added at 50 µL/well, and incubation was performed at 4°C for 30 min. The wells were washed twice with 1% BSA/1×PBS, followed by the addition of PE-conjugated Streptavidin(BD) secondary antibody diluted at a ratio of 1:200, 50 µL per well, and incubation was performed at 4°C for 30 min. After washing twice with 1% BSA/1×PBS, the cells were resuspended in 50 µL of 1% BSA/1×PBS. The cells were analyzed using a flow cytometer (BD Canto II). The results were analyzed using FlowJo software.

The results in Fig. 7 show that the PD-1 antagonistic antibody Keytruda-hIgG1 can significantly block the binding of CHOS-hPD-1 to hPD-L1, while the PD-1 agonistic antibodies P01-hIgG1 and P02-hIgG1 do not block the binding of CHOS-hPD-1 to hPD-L1. Surprisingly, the PD-1 agonistic antibodies 1B5-zu1-hIgG1, 1B5-zu1-hIgG1 DD, 1C4-zu1-hIgG1, and 1C4-zu1-hIgG1 DD not only do not block the binding of CHOS-hPD-1 to hPD-L1, but can even enhance the binding of CHOS-hPD-1 to hPD-L1 to varying degrees. Similarly, the results in Fig. 8 show that Keytruda-hIgG1 can significantly block the binding of CHOS-hPD-1 to hPD-L2, while P01-hIgG1, P02-hIgG1, 1B5-zu1-hIgG1 and 1B5-zu1-hIgG1 DD only weakly inhibit the binding of CHOS-hPD-1 to hPD-L2. Surprisingly, 1C4-zu1-hIgG1 and 1C4-zu1-hIgG1 DD not only do not block the binding of CHOS-hPD-1 to hPD-L2, but can even enhance the binding of CHOS-hPD-1 to hPD-L2 to varying degrees.

### Example 15: Efficacy assay of anti-PD-1 humanized antibodies in a mouse GVHD model

The mouse model of graft-versus-host disease (GVHD) was established using 7-8 week old female immunodeficient mice NOD.CB17-PrkdcscidIl2rgtm1/Bcgen (B-NDG, purchased from Biocytogen, Cat. No. 110586). The modeling method involved injecting 1.5×10⁷ human peripheral blood mononuclear cells (PBMCs) into mice via the tail vein to induce the model. One day after modeling, the antibody to be tested was administered via intraperitoneal injection at a dose of 10 mg/kg, twice a week, for six weeks. During the modeling and drug administration period, the animals' body weight was measured three times a week and their morbidity was monitored. GVHD scores were calculated based on the observation results. When the body weight of the animals was monitored to have decreased by more than 20% compared with the initial body weight, the mice were euthanized.

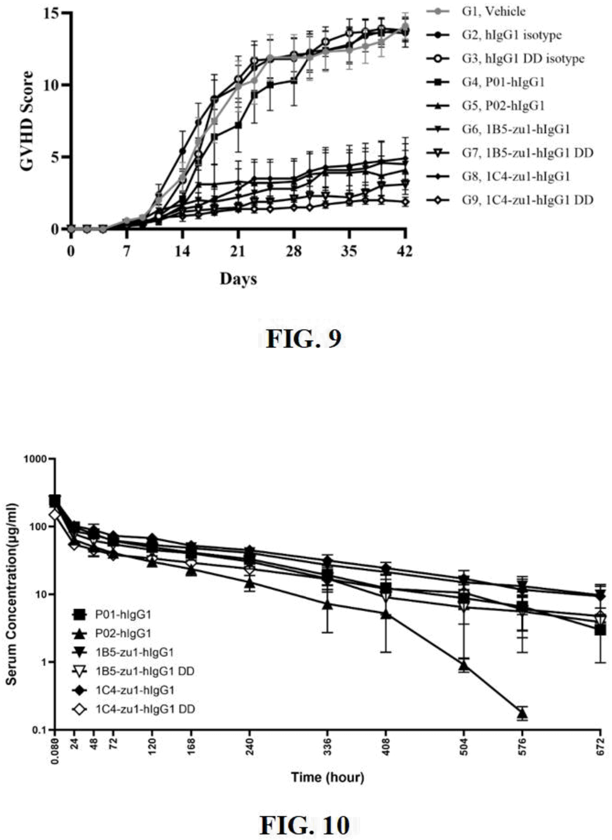

The results are shown in Fig. 9. Compared with the negative control group (Vehicle, hIgG1 isotype, hIgG1 DD isotype), 1B5-zu1-hIgG1, 1C4-zu1-hIgG1, 1B5-zu1-hIgG1 DD, and 1C4-zu1-hIgG1 DD can all significantly alleviate GVHD symptoms in mice, and their efficacy was significantly superior to that of the positive control P01-hIgG1; the efficacy of 1B5-zu1-hIgG1 DD and 1C4-zu1-hIgG1 DD was superior to that of the positive control P02-hIgG1, while the efficacy of 1B5-zu1-hIgG1 and 1C4-zu1-hIgG1 was comparable to that of the positive control P02-hIgG1.

### Example 16: Pharmacokinetic assay of anti-PD-1 humanized antibodies in human FcRn-transgenic mice

Blood samples were collected from human FcRn transgenic mice (purchased from Biocytogen, Cat. No. 110001) at 0h, 0.083h, 24h, 48h, 72h, 120h, 168h, 240h, 336h, 408h, 504h, 576h, and 672h after a single dose to determine the concentration of antibodies present in the serum. The antibody concentration in serum was determined by ELISA. Each group consisted of 6 animals, half male and half female, and the dosage was 10 mg/kg, administered via intravenous injection. Table 14 shows the determined pharmacokinetic properties, where T1/2 represents the half-life, Cmax represents the peak concentration, AUClast represents the area under the curve from the start of administration to the last time point, AUCINF-obs represents the area under the curve from the start of administration to theoretical extrapolation infinity, AUC-%Extrap-obs represents the proportion of the area under the curve from the last point to theoretical extrapolation infinity to AUCINF-obs, Vz-obs represents the apparent volume of distribution, Cl-obs represents the clearance rate, and MRTlast represents the mean residence time.

The results are shown in Fig. 10 and Table 14. 1B5-zu1-hIgG1, 1B5-zu1-hIgG1 DD, 1C4-zu1-hIgG1, and 1C4-zu1-hIgG1 DD all have longer half-lives than P02-hIgG1; 1B5-zu1-hIgG1, 1C4-zu1-hIgG1, and 1C4-zu1-hIgG1 DD have longer half-lives than P01-hIgG1.

**Table 14: Assay results of the pharmacokinetic of anti-PD-1 humanized antibodies in human FcRn-transgenic mice**

| PK Parameter | P01-hIgG1 | P02-hIgG1 | 1B5-zu1-hIgG1 | 1B5-zu1-hIgG1 DD | 1C4-zu1-hIgG1 | 1C4-zu1-hIgG1 DD |
|---|---|---|---|---|---|---|
| T1/2 (hour) | 115.36 | 51.22 | 241.15 | 88.19 | 188.73 | 169.09 |
| Cmax(µg/ml) | 246.29 | 230.58 | 240.02 | 233.27 | 252.81 | 149.43 |
| AUClast(hour*µg /ml) | 20856.12 | 11904.90 | 24817.12 | 18099.31 | 27964.64 | 14983.02 |
| AUCINF-obs(hour*µg/ml) | 21345.24 | 12082.92 | 28472.69 | 18422.86 | 30742.36 | 16057.57 |
| AUC-%Extrap-obs(%) | 2.11 | 1.57 | 12.11 | 1.56 | 8.71 | 6.58 |
| Vz-obs(ml/kg) | 75.01 | 62.98 | 120.65 | 67.57 | 87.47 | 151.87 |
| Cl-obs (ml/hour/kg) | 0.48 | 0.84 | 0.36 | 0.56 | 0.33 | 0.63 |
| MRTlast(hour) | 154.24 | 99.87 | 200.76 | 142.79 | 195.52 | 194.20 |

### Example 17: Efficacy assay of anti-PD-1 humanized antibodies in an SLE mouse model induced by lymphocyte adoptive transfer

The SLE mouse model induced by lymphocyte adoptive transfer is characterized by the production of a series of autoantibodies, including anti-histone and anti-dsDNA antibodies, similar to the disease manifestations observed in human SLE. Seventy male BM12 mice (Jackson Laboratory, Strain No. 001162) were randomly divided into 5 groups (N=8) based on their body weight at 8 weeks of age. All mice received intraperitoneal injections of 5x10⁷ lymphocytes from hPD-1 C57BL/6 donor mice (6-8 weeks old, GemPharmatech, Strain No. T003095), and the experiment lasted for 25 days. Dosing began on D1, with a dosing frequency of twice a week. Blood samples were collected on days 0, 14, and 25 to separate serum and detect anti-dsDNA and anti-histone levels.

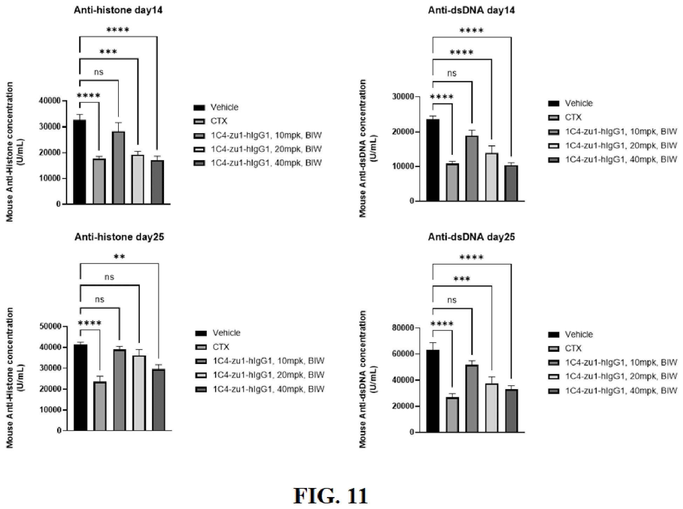

As shown in Fig. 11, on day 14 after lymphocyte adoptive transfer: 1) compared with the negative control group (Vehicle), 1C4-zu1-hIgG1 20mpk and 40mpk significantly reduced the level of anti-histone antibodies; 2) compared with the negative control group (Vehicle), 1C4-zu1-hIgG1 20mpk and 40mpk significantly reduced the levels of anti-dsDNA antibodies. On day 25 after lymphocyte adoptive transfer: 1) compared with the negative control group (Vehicle), 1C4-zu1-hIgG1 40mpk significantly reduced the level of anti-histone antibodies; 2) compared with the negative control group (Vehicle), 1C4-zu1-hIgG1 20mpk and 40mpk significantly reduced the levels of anti-dsDNA antibodies. CTX (cyclophosphamide) served as a positive control in this model.

### Example 18: Efficacy assay of anti-PD-1 humanized antibodies in a TNBS-induced IBD mouse model

Female hPD-1 BALB/c mice (6-8 weeks old, GemPharmatech, Strain No. T002726) were randomly divided into 5 groups (N=10) according to body weight: 1 blank control group (sham group) and 4 TNBS-induced model groups. Immunological intervention was performed on day 0 as follows: mice in the 4 model groups were administered 0.1 mL of 1.5% TNBS solution via rectal instillation; mice in the sham group were administered 0.1 mL of 50% ethanol solution via rectal instillation. The 1C4-zu1-hIgG1 treatment group received intraperitoneal injections on day 1, day 2, and day 5, respectively. Mice in the sham group and the vehicle group (day-1 - day 6) were injected intraperitoneally with the same volume of vehicle excipient. During the experiment, the animals were weighed daily, and the characteristics of mouse feces and occult blood were scored. The disease activity index (DAI) was also calculated. At the experimental endpoint (i.e., day 6), the animals were euthanized. The colon (from the anus to the cecum) of the mice in every group was harvested, the surrounding adipose and connective tissues were removed, and the colon was photographed and measured for length. Subsequently, the contents and cecum were removed, and the colon was weighed.

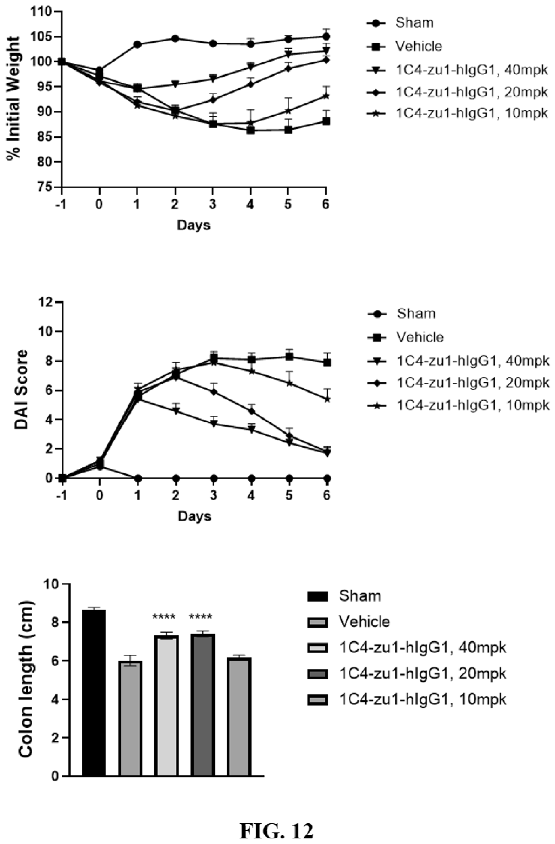

As shown in Fig. 12, 1C4-zu1-hIgG1 has good efficacy in the TNBS-induced acute colitis model in mice. It can alleviate the weight loss and DAI score increase caused by colitis in mice. 1C4-zu1-hIgG1 can significantly alleviate the colonic shortening caused by colitis.

Although the specific embodiments of the present invention have been described in detail, those skilled in the art will understand that various modifications and changes may be made to the details based on all the teachings published, and these changes are all within the scope of protection of the present invention. The entire scope of the present invention is defined by the appended claims and any equivalents thereof.

## Claims

1. An antibody capable of specifically binding to PD-1 or an antigen-binding fragment thereof, comprising:
(a) a VH CDR1 or a variant thereof, a VH CDR2 or a variant thereof, and a VH CDR3 or a variant thereof comprised in a heavy chain variable region (VH) set forth in SEQ ID NO: 7 or 15; and/or a VL CDR1 or a variant thereof, a VL CDR2 or a variant thereof, and a VL CDR3 or a variant thereof comprised in a light chain variable region (VL) set forth in SEQ ID NO: 8 or 16; or
(b) a VH CDR1 or a variant thereof, a VH CDR2 or a variant thereof, and a VH CDR3 or a variant thereof comprised in a heavy chain variable region (VH) set forth in SEQ ID NO: 31 or 39; and/or a VL CDR1 or a variant thereof, a VL CDR2 or a variant thereof, and a VL CDR3 or a variant thereof comprised in a light chain variable region (VL) set forth in SEQ ID NO: 32 or 40;
wherein the variant has one or several amino acid substitutions, deletions or additions (e.g., 1, 2 or 3 amino acid substitutions, deletions or additions, such as conservative substitutions) compared to the sequence from which the variant is derived; preferably, the substitutions are conservative substitutions;
preferably, the antibody or the antigen-binding fragment thereof comprises:
(a) three CDRs comprised in a heavy chain variable region (VH) set forth in SEQ ID NO: 7 or 15; and/or three CDRs comprised in a light chain variable region (VL) set forth in SEQ ID NO: 8 or 16; or
(b) three CDRs comprised in a heavy chain variable region (VH) set forth in SEQ ID NO: 31 or 39; and/or three CDRs comprised in a light chain variable region (VL) set forth in SEQ ID NO: 32 or 40.

2. The antibody or the antigen-binding fragment thereof according to claim 1, comprising:
(a) a heavy chain variable region (VH) comprising the following three complementary determining regions (CDRs): a VH CDR1 set forth in SEQ ID NO: 1, a VH CDR2 set forth in SEQ ID NO: 2, and a VH CDR3 set forth in SEQ ID NO: 3; and/or a light chain variable region (VL) comprising the following three complementary determining regions (CDRs): a VL CDR1 set forth in SEQ ID NO: 4, a VL CDR2 set forth in SEQ ID NO: 5, and a VL CDR3 set forth in SEQ ID NO: 6; or,
(b) a heavy chain variable region (VH) comprising the following three complementary determining regions (CDRs): a VH CDR1 set forth in SEQ ID NO: 25, a VH CDR2 set forth in SEQ ID NO: 26, and a VH CDR3 set forth in SEQ ID NO: 27; and/or a light chain variable region (VL) comprising the following three complementary determining regions (CDRs): a VL CDR1 set forth in SEQ ID NO: 28, a VL CDR2 set forth in SEQ ID NO: 29, and a VL CDR3 set forth in SEQ ID NO: 30;
wherein the CDR is defined by the Kabat numbering system.

3. The antibody or the antigen-binding fragment thereof according to claim 1 or 2, comprising:
(a) a heavy chain variable region (VH) comprising a sequence as set forth in SEQ ID NO: 7 or a variant thereof; and/or a light chain variable region (VL) comprising a sequence as set forth in SEQ ID NO: 8 or a variant thereof; or,
(b) a heavy chain variable region (VH) comprising a sequence as set forth in SEQ ID NO: 31 or a variant thereof; and/or a light chain variable region (VL) comprising a sequence as set forth in SEQ ID NO: 32 or a variant thereof;
wherein the variant has one or several amino acid substitutions, deletions or additions (e.g., 1, 2, 3, 4, or 5 amino acid substitutions, deletions or additions), or a sequence with at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived; preferably, the substitutions are conservative substitutions;
preferably, the antibody or the antigen-binding fragment thereof comprises: a VH comprising a sequence as set forth in SEQ ID NO: 7 and a VL comprising a sequence as set forth in SEQ ID NO: 8;
preferably, the antibody or the antigen-binding fragment thereof comprises: a VH comprising a sequence as set forth in SEQ ID NO: 31 and a VL comprising a sequence as set forth in SEQ ID NO: 32.

4. The antibody or the antigen-binding fragment thereof according to claim 1 or 2, wherein the antibody or the antigen-binding fragment thereof comprises a framework region sequence derived from a human immunoglobulin;
preferably, the antibody or the antigen-binding fragment thereof comprises: a heavy chain framework region of a human heavy chain germline sequence, and/or a light chain framework region of a human light chain germline sequence.

5. The antibody or the antigen-binding fragment thereof according to claim 4, wherein the antibody or the antigen-binding fragment thereof comprises:
(a) a heavy chain variable region (VH) comprising a sequence as set forth in SEQ ID NO: 15 or a variant thereof; and/or a light chain variable region (VL) comprising a sequence as set forth in SEQ ID NO: 16 or a variant thereof; or,
(b) a heavy chain variable region (VH) comprising a sequence as set forth in SEQ ID NO: 39 or a variant thereof; and/or a light chain variable region (VL) comprising a sequence as set forth in SEQ ID NO: 40 or a variant thereof;
wherein the variant has one or several amino acid substitutions, deletions or additions (e.g., 1, 2, 3, 4, or 5 amino acid substitutions, deletions or additions), or has at least 80%, at least 85%, at least 90%, at least 91%, at least 92%, at least 93%, at least 94%, at least 95%, at least 96%, at least 97%, at least 98%, at least 99%, or 100% sequence identity compared to the sequence from which the variant is derived;
preferably, the substitutions are conservative substitutions;
preferably, the antibody or the antigen-binding fragment thereof comprises:
(a) a heavy chain variable region (VH) comprising a sequence as set forth in SEQ ID NO: 15; and/or a light chain variable region (VL) comprising a sequence as set forth in SEQ ID NO: 16; or,
(b) a heavy chain variable region (VH) comprising a sequence as set forth in SEQ ID NO: 39; and/or a light chain variable region (VL) comprising a sequence as set forth in SEQ ID NO: 40.

6. The antibody or the antigen-binding fragment thereof according to any one of claims 1-5, wherein the antibody or the antigen-binding fragment thereof further comprises a constant region derived from a mammal (e.g., human or mouse) immunoglobulin;
preferably, a heavy chain of the antibody or the antigen-binding fragment thereof comprises a heavy chain constant region derived from a human immunoglobulin (such as IgG, e.g., IgG1, IgG2, IgG3, or IgG4), and/or a light chain of the antibody or the antigen-binding fragment thereof comprises a light chain constant region derived from a human immunoglobulin (e.g., κ or λ).

7. The antibody or the antigen-binding fragment thereof according to claim 6, wherein the antibody or the antigen-binding fragment thereof comprises a variant of the human immunoglobulin heavy chain constant region, which has enhanced binding activity to Fc receptors (e.g., CD32A, CD32B and/or CD16A) compared with the wild-type human immunoglobulin heavy chain constant region;
preferably, the antibody or the antigen-binding fragment thereof comprises a human IgG1 or IgG4 heavy chain constant region variant, the heavy chain constant region variant comprises a substitution at one or more of the following positions numbered according to EU: 239, 268; preferably, the substitution is selected from S239D/H268D or S239D/Q268D.

8. The antibody or the antigen-binding fragment thereof according to claim 6 or 7, wherein the antibody or the antigen-binding fragment thereof comprises:
(1) a heavy chain comprising a sequence as set forth in SEQ ID NO: 11 and a light chain comprising a sequence as set forth in SEQ ID NO: 12;
(2) a heavy chain comprising a sequence as set forth in SEQ ID NO: 19 and a light chain comprising a sequence as set forth in SEQ ID NO: 20;
(3) a heavy chain comprising a sequence as set forth in SEQ ID NO: 23 and a light chain comprising a sequence as set forth in SEQ ID NO: 20;
(4) a heavy chain comprising a sequence as set forth in SEQ ID NO: 35 and a light chain comprising a sequence as set forth in SEQ ID NO: 36;
(5) a heavy chain comprising a sequence as set forth in SEQ ID NO: 43 and a light chain comprising a sequence as set forth in SEQ ID NO: 44; or,
(6) a heavy chain comprising a sequence as set forth in SEQ ID NO: 47 and a light chain comprising a sequence as set forth in SEQ ID NO: 44.

9. The antibody or the antigen-binding fragment thereof according to any one of claims 1-8, wherein the antigen-binding fragment is selected from Fab, Fab', F(ab')₂, Fd, Fv, disulfide-linked Fv, scFv, di-scFv, (scFv)₂, a diabody, and a single domain antibody (sdAb); and/or the antibody is a murine antibody, a humanized antibody, a chimeric antibody, a bispecific antibody, or a multispecific antibody.

10. An isolated nucleic acid molecule encoding the antibody or the antigen-binding fragment thereof according to any one of claims 1-9, or the heavy chain variable region and/or the light chain variable region thereof, or the heavy chain and/or the light chain thereof.

11. A vector, comprising the isolated nucleic acid molecule according to claim 10; preferably, the vector is a cloning vector or an expression vector.

12. A host cell, comprising the isolated nucleic acid molecule according to claim 10 or the vector according to claim 11.

13. A method for preparing the antibody or the antigen-binding fragment thereof according to any one of claims 1-9, comprising: culturing the host cell of claim 12 under conditions allowing the expression of the antibody or the antigen-binding fragment thereof, and recovering the antibody or the antigen-binding fragment thereof from the cultured host cell culture.

14. A bispecific or multispecific molecule, comprising the antibody or the antigen-binding fragment thereof according to any one of claims 1-9;
preferably, the bispecific or multispecific molecule specifically binds to PD-1, and additionally specifically binds to one or more other targets;
preferably, the bispecific or multispecific molecule further comprises at least one molecule (e.g., a second antibody) with a second binding specificity for a second target.

15. An immunoconjugate, comprising the antibody or the antigen-binding fragment thereof according to any one of claims 1-9 and a therapeutic agent linked to the antibody or the antigen-binding fragment thereof;
preferably, the therapeutic agent is a cytotoxic agent.

16. A pharmaceutical composition, comprising the antibody or the antigen-binding fragment thereof according to any one of claims 1-9, the isolated nucleic acid molecule according to claim 10, the vector according to claim 11, the host cell according to claim 12, the bispecific or multispecific molecule according to claim 14 or the immunoconjugate according to claim 15, and a pharmaceutically acceptable carrier and/or excipient;
preferably, the pharmaceutical composition further comprises an additional pharmaceutically active agent;
preferably, the additional pharmaceutically active agent is an immunosuppressant;
preferably, the additional pharmaceutically active agent is a drug for treating autoimmune diseases, allergic diseases, graft-versus-host disease, transplant rejection, or lymphoma.

17. Use of the antibody or the antigen-binding fragment thereof according to any one of claims 1-9, the isolated nucleic acid molecule according to claim 10, the vector according to claim 11, the host cell according to claim 12, the bispecific or multispecific molecule according to claim 14, the immunoconjugate according to claim 15 or the pharmaceutical composition according to claim 16 in the preparation of PD-1 signaling pathway activators.

18. Use of the antibody or the antigen-binding fragment thereof according to any one of claims 1-9, the isolated nucleic acid molecule according to claim 10, the vector according to claim 11, the host cell according to claim 12, the bispecific or multispecific molecule according to claim 14, the immunoconjugate according to claim 15 or the pharmaceutical composition according to claim 16 in the preparation of immunosuppressants (such as T-cell activity inhibitors).

19. Use of the antibody or the antigen-binding fragment thereof according to any one of claims 1-9, the isolated nucleic acid molecule according to claim 10, the vector according to claim 11, the host cell according to claim 12, the bispecific or multispecific molecule according to claim 14, the immunoconjugate according to claim 15 or the pharmaceutical composition according to claim 16 in the preparation of a drug, wherein the drug is used for preventing and/or treating autoimmune diseases, allergic diseases, graft-versus-host disease, transplant rejection, or lymphoma in a subject;
preferably, the autoimmune disease is selected from arthritis, rheumatoid arthritis, psoriatic arthritis, lupus, lupus nephritis, systemic lupus erythematosus, psoriasis, vitiligo, alopecia areata, inflammatory bowel disease, ulcerative colitis, Crohn's disease, type I diabetes, multiple sclerosis, autoimmune hepatitis, primary biliary cirrhosis, celiac disease, scleroderma, Graves' disease, Hashimoto's thyroiditis, ankylosing spondylitis, myasthenia gravis, Sjögren's syndrome, IgA nephropathy, IgG4-related disease, vasculitis, ANCA-associated vasculitis, uveitis, pemphigus, pemphigoid, or autoimmune hemolytic anemia;
preferably, the allergic disease is selected from idiopathic thrombocytopenic purpura, atopic dermatitis, asthma, allergic rhinitis, drug allergy, food allergy, anaphylaxis, allergic conjunctivitis, urticaria, eosinophilic sinusitis, eosinophilic gastrointestinal disease, or allergic bronchopulmonary aspergillosis;
preferably, the lymphoma is T-cell lymphoma;
preferably, the subject is a mammal, such as a human or a non-human primate (e.g., cynomolgus monkey);
preferably, the antibody or the antigen-binding fragment thereof is used alone or in combination with an additional pharmaceutically active agent; preferably, the additional pharmaceutically active agent is an immunosuppressant; preferably, the additional pharmaceutically active agent is a drug for treating autoimmune diseases, allergic diseases, graft-versus-host disease, transplant rejection, or lymphoma.

20. A conjugate, comprising the antibody or the antigen-binding fragment thereof according to any one of claims 1-9 and a detectable label linked to the antibody or the antigen-binding fragment thereof;
preferably, the detectable label is selected from an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

21. Use of the antibody or the antigen-binding fragment thereof according to any one of claims 1-9 or the conjugate according to claim 20 in the preparation of a detection reagent, wherein the detection reagent is used for detecting the presence or level of PD-1 in a sample and/or diagnosing a PD-1-related disease;
preferably, the PD-1-related disease includes autoimmune disorders, allergic diseases, graft-versus-host disease, transplant rejection, lymphoma, or any combination thereof;
preferably, the PD-1-related disease is selected from tumors.
